# EUROPEAN PATENT SPECIFICATION

(11) **EP 2 155 294 B1**
(45) Date of publication and mention of the grant of the patent: **15.02.2012**
(21) Application number: 08768479.1
(22) Date of filing: 13.06.2008
(51) Int. Cl.: A61M 5/145, A61M 5/165, A61M 5/178, A61M 5/31, A61J 1/20

(54) **METHODS AND DEVICES FOR MINIMALLY-INVASIVE DELIVERY OF CELL-CONTAINING FLOWABLE COMPOSITIONS**
VERFAHREN UND VORRICHTUNGEN ZUR MINIMAL INVASIVEN ABGABE VON ZELLHALTIGEN FLIESSFÄHIGEN ZUSAMMENSETZUNGEN
PROCÉDÉS ET DISPOSITIFS DE DISTRIBUTION MINIMALEMENT INVASIVE DE COMPOSITIONS LIQUIDES CONTENANT DES CELLULES

(30) Priority: 13.06.2007 US 934385 P
(43) Date of publication of application: 24.02.2010
(73) Proprietor: Pervasis Therapeutics, Inc., Cambridge, Massachusetts 02139 (US)
(72) Inventor: KANNER, Glenn, Duxbury, MA 02332 (US); BOLLINGER, Stephen, August, Mansfield, MA 02048 (US); NUGENT, Helen, Marie, Needham, MA 02492 (US); CHOI, Celina, Brighton, MA 02135 (US); WHITE, Desmond, Oxford, MA 01540 (US); NG, Yin, Shan, North Billerica, MA 01862 (US)
(74) Representative: Lock, Graham James
(86) International application number: PCT/US2008/007454
(87) International publication number: WO 2008/156705

(56) References cited:
- WO-A-2005/120431
- WO-A-2006/120461
- US-A1- 2007 088 252
- US-A1- 2007 106 244

## Description

### Background

There are many anatomical structures within the body which are subject to injury, damage or disease. These include, for example, tubular structures such as arteries and veins, the esophagus, stomach, small and large intestine, biliary tract, ureter, bladder, urethra, nasal passageways, trachea and other airways, and the male and female reproductive tract. These also include the interior and/or exterior surface of solid tissues and organs such as the kidney, liver, lung, bone, nerve, resected tumor sites, stroma, heart and muscle. Injury, various surgical procedures or disease can result in the narrowing, weakening and/or obstruction of such anatomical structures, resulting in serious complications and/or even death.

Existing endoluminal interventions, delivery devices and associated methods involve forceful manipulation within the lumen of a tubular structure such as a blood vessel. These interventions can damage or denude the cell lining of the lumen, which can hasten stenosis and cause lumen wall rupture, and/or plaque formation. Therefore, treatment options for diseases of tubular structures such as blood vessels and for diseases of solid organs or tissues, such as cardiac tissue, and for clinical sequelae such as stenosis, fibrosis and inflammation following therapeutic interventions of tubular and solid tissues continue to be major problems with respect to long-term outcomes for these conditions.

One therapeutic intervention involves the use of cells and/or cell-containing implantable materials. Implanting cells and/or a cell-containing flowable composition in a patient, however, poses numerous challenges. For instance, the cells and/or the cell-containing flowable composition must endure the stresses caused by delivery, manipulation at the site of administration, and/or pressure exerted by surrounding tissue, for example, while at the same time maintaining cell viability, cell functionality and matrix integrity. Delivery by, for example, needle passage, may expose a cell or a cell-containing flowable composition to certain frictional and/or shearing forces as well as injection and/or tissue pressures. Moreover, the cells or cell-containing flowable composition may need to be shaped at the site of administration or sequestered at the site of administration in certain clinical applications. Further, injection pressures or tissue pressures at the site of administration may damage the cells or the cell-containing flowable composition or may cause the cells or the cell-containing flowable composition to shift to a location other than the optimal site of administration.

Additionally, the cells and/or the cell containing implantable materials must endure the stresses caused by transport to the location of the therapy, extended periods of shelf-life prior to use, preparatory procedures, and transition to a delivery device, for example, while at the same time maintaining cell viability, cell functionality and matrix integrity. Similar to the stresses caused by delivery of the material to an anatomic location, delivery of the material to a transport container or a delivery device, for example, may expose a cell or a cell-containing flowable composition to certain frictional and/or shearing forces as well as injection pressures.

It is an objective of the present invention to provide methods and devices for transport, preparation for implantation, and minimally-invasive localized delivery of cells, for example as a cell-containing implantable material, for treatment of tubular body parts as well as other solid tissues, organs, resection sites and stroma.

### Summary of Invention

The present invention provides a device as defined in the claims.

### Brief Description of the Drawings

Figure 1 is a graphical illustration of the flow rate of a particulate biocompatible material passed through needles of variable internal diameters.

Figure 2 is a graphical illustration of a microcarrier biocompatible material passed through a variety of needles having variable internal diameters.

Figure 3 is a graphical illustration of the flow rate of a biocompatible material at a variety of particle concentrations passed through a 30 gauge needle at 80 psig.

Figure 4 is a perspective view of an illustrative delivery device according to one embodiment of the present invention.

Figure 5 is a perspective view of an illustrative marker device according to one embodiment of the present invention.

Figure 6 is a perspective view of an illustrative guidance device according to one embodiment of the present invention.

Figures 7A, 7B, 7C and 7D illustrate a series of steps according to an illustrative method of delivery using an illustrative delivery, marker and guidance devices of Figures 4, 5 and 6 according to one embodiment of the present invention.

Figure 8 is a perspective view of an illustrative dispensing container according to one embodiment of the present invention.

Figure 9 is a cut-away view of the illustrative dispensing container of Figure 8 according to one embodiment of the present invention.

Figures 10A and 10B are cut-away views of the illustrative dispensing container of Figure 8 according to one embodiment of the present invention.

Figure 11 is a cut-away view of the illustrative dispensing container of Figure 8 according to one embodiment of the present invention.

Figures 12A and 12B are cut-away views of the illustrative dispensing container of Figure 8 according to one embodiment of the present invention.

Figures 13A and 13B are cut-away views of the illustrative dispensing container of Figure 8 according to one embodiment of the present invention.

Figures 14A and 14B are cut-away views of the illustrative dispensing container of Figure 8 according to one embodiment of the present invention.

Figures 15A and 15B are cut-away views of the illustrative dispensing container of Figure 8 according to one embodiment of the present invention.

Figures 16A and 16B are cut-away views of the illustrative dispensing container of Figure 8 according to one embodiment of the present invention.

Figure 17A is a perspective view of an illustrative delivery syringe according to one embodiment of the present invention.

Figure 17B is a perspective view of an illustrative delivery syringe and needle according to one embodiment of the present invention.

Figure 18A is a cut-away view and Figure 18B is a perspective view of the illustrative delivery syringe of Figure 17A according to one embodiment of the present invention.

Figure 19A is a cut-away view and Figure 19B is a perspective view of the illustrative delivery syringe of Figure 17A according to one embodiment of the present invention.

Figure 20A is a cut-away view and Figure 20B is a perspective view of the illustrative delivery syringe of Figure 17A according to one embodiment of the present invention.

Figure 21A is a cut-away view of the illustrative delivery syringe of Figure 17A and Figure 21B is a perspective view of the illustrative delivery syringe with needle of Figure 17B according to one embodiment of the present invention.

Figure 22A is a cut-away view of the illustrative delivery syringe of Figure 17A and Figure 22B is a perspective view of the illustrative delivery syringe with needle of Figure 17B according to one embodiment of the present invention.

### Detailed Description of the Invention

The present invention exploits the discovery that cells and certain cell-containing flowable compositions can be disposed in a minimally-invasive manner on an exterior or non-luminal surface of a tubular body part or an interior or exterior surface or volume of a solid organ or tissue and can effectuate healing of the interior lumen or other targeted surface and restore homeostasis of the anatomical structure. The invention finds application in methods for delivering cells and/or cell-containing flowable compositions to treat, ameliorate, manage and/or reduce the progression of various diseases and traumas without diminishing the clinical effectiveness or the viability of the cells. The teachings presented below provide sufficient guidance to make and use the devices and methods of the present invention, and further provide sufficient guidance to identify suitable criteria and parameters for testing, measuring, and monitoring the performance of the materials and methods of the present invention.

Using the minimally-invasive devices and methods disclosed herein, cells and cell-containing flowable compositions can be applied to any anatomical structure requiring interventional therapy to maintain homeostasis. As contemplated herein, anatomical structures are those having an interior surface, for example and interior luminal surface, an exterior or extraluminal surface or non-luminal components, for example, solid tissue or organ structures. In certain tubular structures, the interior luminal surface is an endothelial cell layer. In certain other tubular structures, the interior luminal surface is a non-endothelial cell layer. The present invention is effective to treat an endothelial-lined or non-endothelial-lined tubular structure. In certain other solid organ or tissue structures, the interior and/or exterior surface or volume is an endothelial cell layer. In certain other solid organ or tissue structures, the interior and/or exterior surface or volume is a non-endothelial cell layer. For purposes of the present invention, an exterior or extraluminal surface can be, but is not limited to, an exterior surface of a tubular or non-tubular tissue or organ structure. Furthermore, non-luminal components can be, but are not limited to, adventitial, medial, and interstitial components of a tubular or non-tubular tissue or organ structure. It is understood that non-luminal components can also include the space between the tubular or non-tubular structure and the surrounding sheath or connective tissue, the exterior surface of the surrounding sheath or connective tissue, or on the surface or within a volume of adjacent muscle tissue. For example, in the case of a blood vessel, non-luminal components can include, for example, the space between the exterior surface of the blood vessel and the interior surface of the vascular sheath, the exterior surface of the vascular sheath, the space between the muscular sheath and the adjacent muscle, or within the adjacent muscle tissue. It is further contemplated that administration of the implantable material at or to the interior surface or volume of a solid tissue or organ includes administration into the tissue or organ, for example, by direct injection. The tissue or organ need not have a defined interior surface for administration.

Tubular anatomical structures include structures of the vascular system, the reproductive system, the genitourinary system, the gastrointestinal system, the pulmonary system, the respiratory system and the ventricular system of the brain and spinal cord. Representative examples of tubular anatomical structures include arteries and veins, lacrimal ducts, the trachea, bronchi, bronchiole, nasal passages (including the sinuses) and other airways, eustachian tubes, the external auditory canal, oral cavities, the esophagus, the stomach, the duodenum, the small intestine, the large intestine, biliary tracts, the ureter, the bladder, the urethra, the fallopian tubes, uterus, vagina and other passageways of the female reproductive tract, the vas deferens and other passages of the male reproductive tract, a vascular sheath, and the ventricular system (cerebrospinal fluid) of the brain and spinal cord. For purposes of the present invention, tubular anatomical structures can be naturally occurring or non-naturally occurring.

Interventions of vascular tubular structures result in vascular injuries susceptible to treatment with the present invention. Exemplary interventions include but are not limited to angioplasty, atherectomy, vascular stenting including bare-metal and drug-eluting stents, vascular bypass surgeries including arterial bypass grafts and peripheral bypass grafts, organ transplantation, arteriovenous fistula and other vascular anastomosis formation, arteriovenous, peripheral and other graft formation, and subsequent vascular access-associated injuries, including needle sticks incurred during accessing a vessel for dialysis or other interventional therapy. Each vascular intervention results in a degree of injury to the endothelial cell lining of the vascular lumen. In turn, the injured vascular lumen experiences a cascade of biochemical events resulting in a variety of clinically identifiable sequelae, including but not limited to occlusive thrombosis, stenosis, intimal hyperplasia, acute and chronic inflammation, fibroblast and smooth muscle cell proliferation and migration, vascular remodeling, adventitial fibrosis and neovascularization, vasodilation and the formation of vulnerable plaque lesions.

Intervetions of non-vascular tubular structures result in injuries susceptible to treatment with the present invention. Exemplary interventions include but are not limited to diseases or disorders of the tubular structure or surgical intervention of the tubular structure resulting in one or more of a variety of clinically identifiable sequelae, including but not limited to acute and chronic inflammation, tissue remodeling, occlusive cell proliferation, dilation and the formation of lesions on, in or within the tubular structure.

The cells or the cell-containing flowable composition can be applied to the exterior surface of soft tissue structures or organs requiring interventional therapy to maintain homeostasis. Soft tissue structures include, for example, muscle, connective tissue, fat, vasculature, and peripheral nerves. Organs include, for example, the heart, lungs, kidneys, uterus, pancreas, liver, or spleen. As contemplated herein, organs are tissue structures having an exterior and/or an interior volume or surface.

The cells or the cell-containing flowable composition can be applied at the time of primary treatment of the injury, damage or disease or at the time of surgical or other intervention. The cells or the cell-containing flowable composition can be applied at a time following the primary treatment or intervention, for example, as a secondary treatment or continuing-care treatment. Secondary treatments can be administered monthly, bimonthly, or at another time period determined by a physician based on the injury, damage or disease being treated.

### Cells and Cell-Containing Flowable Compositions

The devices of the present invention may be adapted for use with implantable flowable cells and/or cell-containing flowable compositions, also referred to herein as a flowable composition, which are more fully discussed in International Publication No. WO 2006/062871.

Briefly, International Publication No. WO 2006/062871 discloses a flowable cell-containing composition for treating an injured or diseased site in a subject wherein the flowable composition comprises a biocompatible matrix and cells, wherein the flowable composition is in an amount effective to treat the injured or diseased site. The biocompatible matrix can be a particle, a gel, a foam, or a suspension. The biocompatible matrix can comprise particles and/or microcarriers. The particles and/or microcarriers can further comprise gelatin, collagen, fibronectin, fibrin, laminin, dextran, cellulose, polylactic acid (PLA), polyglycolic acid (PGA), poly(ethylene-co-methacrylic acid) (PEMA), or an attachment peptide. One exemplary attachment peptide is a peptide of sequence arginine-glycine-aspartate (RGD). According to a preferred embodiment, the particle or microcarrier has a diameter of about 20 microns to about 500 microns, preferably a diameter of about 200 microns.

The flowable composition may further comprise a carrier fluid. The flowable composition may comprise cells, cells and an attachment peptide or cells and a carrier fluid without a biocompatible matrix. The flowable composition may be shape-retaining, thereby permitting the clinician to control the amount of and location of deposition to an extent necessary given a particular site of administration.

A single dose of the flowable composition may comprise approximately 0.5 x 10⁶ to 3 x 10⁶ cells in a total volume of approximately 2 cubic centimeters of flowable composition. If the biocompatible material is a gelatin particulate material, a single dose comprises approximately 75 mg particles and a.final concentration of about 10-50 mg/ml flowable composition. If the biocompatible material is a gelatin microcarrier material, a single dose comprises approximately 10-20 mg microcarrier beads and a final concentration of about 10-50 mg/ml flowable composition. A single dose can comprise approximately 5-500 mg of flowable composition, more preferrably 10-200 mg, and most preferrably 20-60 mg, administered in a single dose or in multiple sequential doses.

Microcarrier beads often settle in a group, and so the microcarrier-based flowable composition may result in a smaller relative site of deposition. Accordingly, depending on the desired site of administration, multiple doses of the microcarrier-based flowable composition may be required to cover the desired site of administration with the flowable composition. However, because the microcarrier beads contain a higher concentration of cells, relative to the particle-based flowable composition, a single dose of the microcarrier-based flowable composition is able to deliver a therapeutic dosage of cells in a relatively smaller volume.

The implantable flowable composition may comprise cells, such as, for example, endothelial cells, endothelial-like cells, epithelial cells, epithelial-like cells, or non-endothelial cells. In a preferred embodiment, endothelial cells are obtained from vascular tissue, preferably but not limited to arterial tissue. Sources of vascular endothelial cells suitable for use include aortic endothelial cells, umbilical cord vein endothelial cells, saphenous vein endothelial cells, coronary artery endothelial cells, pulmonary artery endothelial cells and iliac artery endothelial cells. The composition can comprise one cell type, or two or more different cell types. Cells can be allogeneic, xenogeneic or autologous. Cells can be selected on the basis of their tissue source and/or their immunogenicity. A source of living cells can be derived from a suitable donor or multiple donors, or derived from a cadaver or from a cell bank.

The cell-containing flowable composition described herein comprises cells engrafted on, in, and/or within a biocompatible matrix. Engrafted means securedly attached via cell to cell and/or cell to matrix interactions such that the cells withstand the rigors of the preparatory and delivery manipulations disclosed herein. The cell-containing flowable composition may comprise near-confluent, confluent or post-confluent cell population having a preferred phenotype. It is understood that cells or the cell-containing flowable composition likely shed cells during preparatory manipulations and/or that certain cells are not as securedly attached as are other cells.

The cells of the flowable composition are tested for the presence of one or more preferred phenotypes or functional properties. It has been discovered that a cell having a readily identifiable phenotype when associated with a preferred biocompatible matrix or otherwise in a near-confluent, confluent or post-confluent condition can facilitate, restore, and/or otherwise modulate endothelial, non-endothelial and/or non-luminal cell physiology and/or luminal or adventitial homeostasis associated with the treatment of an injured or diseased tubular or solid tissue structure or a tubular or solid tissue structure subject to surgical or other intervention resulting in damage or disease of the tubular or solid tissue structure. Preferred, readily identifiable phenotypes include, for example, the ability to inhibit or otherwise interfere with excessive cell proliferation and migration, tissue modeling, and/or acute and chronic inflammation or fibrosis.

It is understood that the presently disclosed delivery devices and methods of using the same are effective to deliver cells with or without an implantable, flowable substrate admixed with the cells, or upon which the cells have been grown. The teachings provided herein are not limited to cell-containing flowable compositions. The skilled artisan will readily appreciate that preparations of cells per se are deliverable using the devices and methods disclosed herein and use of cells per se only requires routine optimization of the conditions and manipulations taught herein.

### Delivery Devices

As disclosed herein, minimally invasive delivery devices can be used to deliver clinically effective viable cells or the cell-containing flowable composition to a site within a subject in need thereof. An exemplary delivery device comprises a penetration device coupled to a dispensing container for delivering the cells or the cell-containing flowable composition wherein the penetration device is adapted for use with the open field, extraluminal and endoluminal treatment paradigms described herein.

The penetration device of the minimally-invasive delivery device may comprise a needle or needle-like structure having an internal diameter adapted to allow passage and delivery of clinically effective viable cells or the cell-containing flowable composition to an anatomical target site, and an external diameter adapted to allow passage of the penetration device through the route of administration to the site of administration without unreasonably adversely affecting the adjacent tissues. Accordingly, a preferred penetration device includes a relatively thin wall, accommodating a relatively large internal diameter and a relatively smaller external diameter.

The penetration device of the minimally-invasive delivery device may comprise a catheter or a catheter-like structure having an internal diameter adapted to allow passage of clinically effective viable cells or the cell-containing flowable composition to an anatomical target site. The penetration device may comprise a needle-like structure coupled to a catheter-like structure.

The penetration device selected to deliver the flowable composition may have a needle gauge and an internal diameter suited to deliver the clinically effective viable cells or the cell-containing flowable composition of that embodiment of the invention to the desired site of administration. For example, depending on the characteristics of the selected biocompatible material and/or characteristics of the selected formulation of flowable composition and/or the characteristics of the selected route of administration, the flowable composition can have, for example, a variable flowability, a variable concentration of cells and/or a variable concentration of biocompatible material in the flowable composition and/or a variable minimum necessary pressure for administration of the material during delivery. Accordingly, a penetration device can be selected to optimize the administration of a given flowable composition.

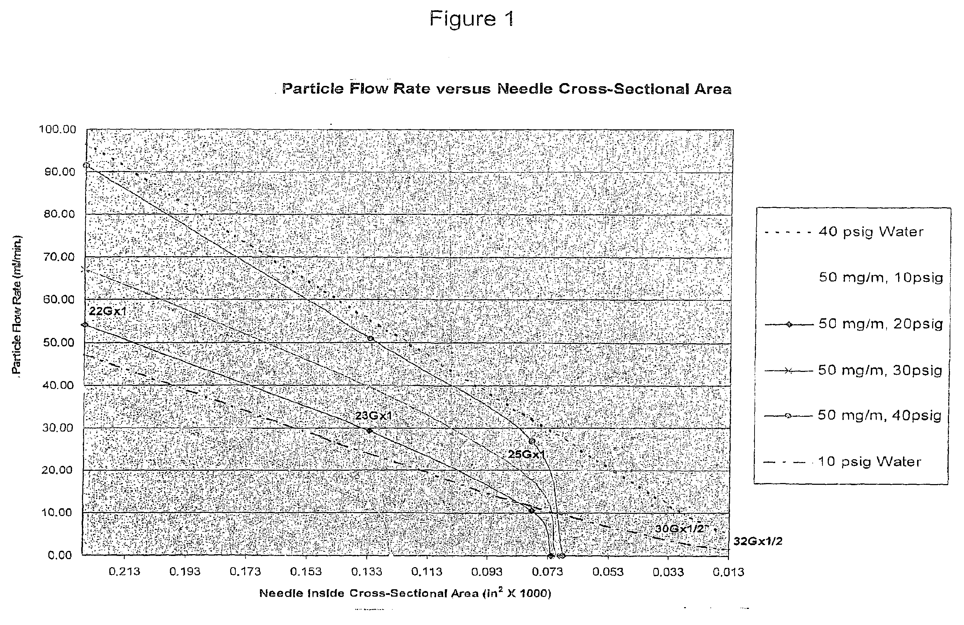

Figure 1 is a graphical illustration of the flow rate of one preferred particulate biocompatible material, wherein, hydrated Gelfoam^{®} particles (Pfizer, New York, NY), passed through a variety of needles having variable internal diameters. With reference to Figure 1, a flowable composition formulation comprising 50 mg/mL Gelfoam particulate matrix hydrated with water can be passed through needles having an internal diameter from 25 gauge to 22 gauge.

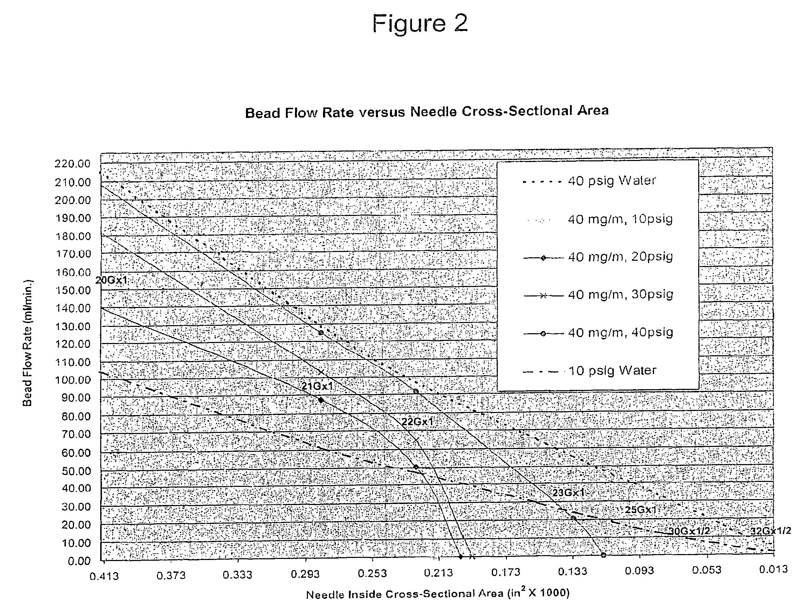

Figure 2 is a graphical illustration of the flow rate of one preferred microcarrier biocompatible material, wherein, hydrated CultiSpher-G™ microcarrier beads (Percell Biolytica AB, Astorp, Sweden), passed through a variety of needles having variable internal diameters. With reference to Figure 2, a flowable composition formulation comprising 40 mg/mL CultiSphere microcarrier bead matrix hydrated with water can be passed through needles having an internal diameter from 23 gauge to 18 gauge.

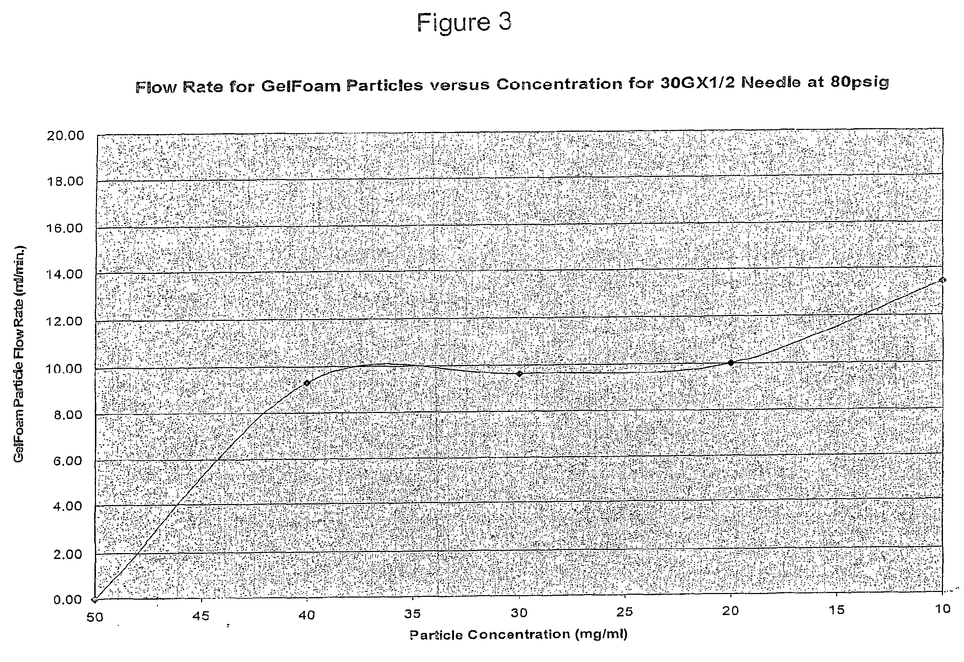

Figure 3 is a graphical illustration of the flow rate of one preferred particulate biocompatible material, wherein, hydrated Gelfoam^{®} particles (Pfizer, New York, NY), at a variety of particle concentrations passed through a 30 gauge needle at 80 psig. With reference to Figure 3, a flowable composition formulation comprising a concentration of 45 mg/mL to 10 mg/mL particulate material hydrated with water can be passed through a 30 gauge needle. Additionally, this data suggests that a particulate composition of 50 mg/mL is sufficiently dense that it is not capable of being passed through a 30 gauge needle at 80 psig. Further, although a particulate composition of 10 mg/mL or 15 mg/mL is capable of being passed through a 30 gauge needle at 80 psig, each of these concentrations requires a larger relative volume of material to achieve a desired cell count or cell number such that these concentrations are less preferred for certain indications.

The flowable composition may comprise a gelatin particulate biocompatible material suited for non-luminal and/or endoluminal administration. In this case, a preferred penetration device is an 24 gauge to an 18 gauge or smaller gauge internal diameter needle or catheter, more preferably a 24 gauge to a 21 gauge internal diameter needle or catheter, most preferably a 24 gauge to a 23 gauge internal diameter needle or catheter. Here, a preferred penetration device is a 25 gauge to a 18 gauge external diameter needle or catheter, more preferably a 25 gauge to a 21 gauge external diameter needle or catheter, most preferably a 25 gauge to a 23 gauge external diameter needle or catheter. Accordingly, penetration devices include needles or catheters with (1) an internal diameter of 24 gauge and an external diameter of 25-23 gauge; (2) an internal diameter of 25 gauge and an external diameter of 25-23 gauge; and (3) an internal diameter of 23 gauge and an external diameter of 25-23 gauge.

The flowable composition may comprise a gelatin microcarrier biocompatible material suited for percutaneous administration. Here, a preferred penetration device has a 23 gauge to an 18 gauge or smaller gauge internal diameter needle, preferably a 22 gauge to an 18 gauge internal diameter needle.

The penetration device may be capable of administering the flowable composition at a pressure insufficient to diminish the therapeutic effectiveness of the material.

The gelatin particulate biocompatible material, in relation to the gelatin microcarrier biocompatible material, has what appears to be a more neutrally-buoyant nature and therefore a higher relative flowability. Accordingly, the particulate material can be delivered using relatively higher gauge (smaller internal diameter) needles and/or catheters. On the contrary, the gelatin microcarrier biocompatible material has what appears to be a relatively higher specific gravity and a relatively higher ability to attach to other similar particles, resulting in settling and clumping of microcarriers or separation of microcarriers from a solution of biocompatible material and therefore a lower relative flowability. Accordingly, the microcarrier material requires a relatively lower gauge (larger internal diameter) delivery needle or catheter. Additionally, because the microcarriers require a relatively lower gauge (larger internal diameter) needle or catheter and because the external diameter of the preferred catheter approaches the internal diameter of most catheter-accessible blood vessels, the preferred penetration device for the flowable composition comprising gelatin microcarriers is a needle. Accordingly, when endoluminal catheter administration is desired, the preferred biocompatible material for the flowable composition is the gelatin particulate material.

The penetration device can permit, for example, a single point of delivery or a plurality of delivery points arranged in a desired geometric configuration to accomplish delivery of the cells or the cell-containing flowable composition without disrupting an injured or diseased target site. A plurality of delivery points can be arranged, for example, in a single circle, concentric circles, or a linear array arrangement to name but a few. In another embodiment, the penetration device comprises a single piercing member, such as, for example, a single needle or a catheter, with a plurality of penetration devices retractably disposed within the piercing member, wherein the cells or the cell-containing flowable composition passes through one or more of the plurality of penetration devices during delivery. The penetration device can also be in the form of a balloon stent including a plurality of delivery points. It is understood that the penetration devices contemplated herein can be configured to administer the cells or the cell-containing flowable composition via percutaneous and/or endoluminal access methods.

The penetration device (*e.g*., a needle) may be in fluid connection with a dispensing container which accommodates a quantity of cells or cell-containing flowable composition for delivery through the penetration device to a non-luminal site of administration in a subject. The dispensing container may be an integral member of the delivery device and is connected to the penetration device, or a separate device adapted to be in fluid communication with the penetration device or other portion of the delivery device. An exemplary dispensing container is described in greater detail below.

The dispensing container stores a sufficient volume of cells or cell-containing flowable composition for a single deposition at a site of administration within a subject in need thereof, or a sufficient volume of cells or cell-containing flowable composition for multiple depositions at multiple sites of administration within a subject in need thereof.

The dispensing container may be suitable for use during any one, more than one, or all of the following flowable composition preparation and administration steps: cell growth, cell expansion, cell seeding onto a biocompatible matrix material, incubation on a biocompatible matrix material, and cell growth to confluence. The dispensing container may additionally support simple media changes during incubation while retaining the cells and the matrix material within the dispensing container. The dispensing container may be further adapted to serve as a transport container during manufacturing and/or a shipping container, or be further suited for use during rinse procedures by end-users prior to administration of the flowable composition. Additionally, the dispensing container can serve as a reservoir for the flowable composition during administration of the flowable composition. Here, the dispensing container can be a part of or integrated into the delivery device.

The delivery device and/or the dispensing container may be preloaded with a sufficient amount of the cells or cell-containing flowable composition prior to or coincident with deposition at the site of administration. A delivery device, such as, for example, a dispensing container integral to or adapted to be in fluid communication with a delivery device, or a penetration device adapted to contain a sufficient volume of the cells or cell-containing flowable composition, may be delivered to a clinical site preloaded with a sufficient amount of the cells or cell-containing flowable composition ready for implantation. Likewise, a delivery device can be delivered to an operator, such as a physician, preloaded with a sufficient amount of the cells or cell-containing flowable composition ready for implantation.

The delivery device can be used in combination with a delivery sheath, when the delivery sheath comprises a tube having a hollow lumen extending laterally along at least a portion of its tength. The delivery device may be slidably disposed within the lumen of the delivery sheath, when the sheath can be retracted, or the delivery device extended, to expose the distal end of the delivery device from the distal end of the sheath for delivery according to a method of the invention. Likewise, the sheath can be extended, or the delivery device retracted, to enclose the distal end of the delivery device for removal of the device from a subject.

The delivery device may be in communication with a control module which allows an operator of the delivery device to modulate the rate and location of administration of the cells or cell-containing flowable composition within the subject. The control module may be a mechanical device, for example a syringe or bulb. The control module may be an electrical device, or a pressurized line, such as a hose, in communication with the penetration device directly or indirectly, for example, across a membrane, diaphragm or piston. The pressure of the pressurized line and the resultant rate of administration can be modulated by the operator of the delivery device during delivery.

The delivery device can be used in conjunction with one or more guidance devices to locate the desired target site of administration within the patient. The guidance device may be intraluminal guidance device, for example, a guidewire, catheter, intravascular ultrasound (IVUS), fluoroscopy or other endoscopic guidance system known in the field, which is inserted intraluminally to the site of administration prior to introduction of the delivery device and which directs the delivery device to the site of administration. The guidance device may be an external guidance device, for example, color Doppler ultrasound, duplex ultrasound, two dimensional ultrasound, B-mode ultrasound, magnetic resonance angiography (MRA), magnetic resonance imaging (MRI), CT scanning, fluoroscopy to identify the location of the target site or of a stent, guidewire or catether, and/or other guidance systems known in the field. The guidance device may be used to identify the target site of administration within the subject.
The guidance device may be used to identify a marker device, described in greater detail below, located at or near the target site to identify the target site of administration within the subject. Additionally, the delivery device can be used in conjunction with tactile palpation or contrast enhancement to located the target site of administration.

The guidance device may comprise ultrasound imaging. Ultrasound imaging does not adversely affect the cells contained within the flowable composition and permits a range of view angles during administration. For example, in the case of administration to a luminal structure, ultrasound imaging permits both longitudinal and cross-sectional views of the site of administration with slight modifications in alignment of the ultrasound device. Here, the penetration device preferrably comprises an echogenic material and/or an echogenic coating, resulting in the penetration device being visible by ultrasound imaging during administration of the material to the desired site. Further, the flowable composition preferably comprises an echogenic material, resulting in the flowable composition being visible by ultrasound imaging during and/or following administration of the material to the desired site. Exemplary echogenic flowable compositions include, but are not limited to, collagen and gelatin. Additionally, an echogenic material can be added to a non-echogenic biocompatible material, resulting in an echogenic flowable composition. The added echogenic material may not adversely affect the viability and/or efficacy of the cells or the flowable composition.

The guidance device may comprise fluoroscopy imaging. Here, the penetration device preferrably comprises a fluoroscopically visible material and/or fluoroscopically visible coating, resulting in the penetration device being visible by fluoroscopic imaging during the admininstration of the material to the desired site. Further, the flowable composition preferrably comprises a fluoroscopic material, resulting in the flowable composition being visible by fluoscopic imaging during and/or following administration of the material to the desired site. Exemplary fluoroscopic flowable compositions include, but are not limited to, a fluorscopic material that can be added to a non-fluroscopic biocompatible material, resulting in a fluoroscopic flowable composition. Exemplary fluoroscopic materials include contrast agent, contrast media, contrast dye, contrast liquid and other fluoroscopically visible material. The added fluoroscopic material may not adversely affect the viability and/or efficacy of the cells or the flowable composition.

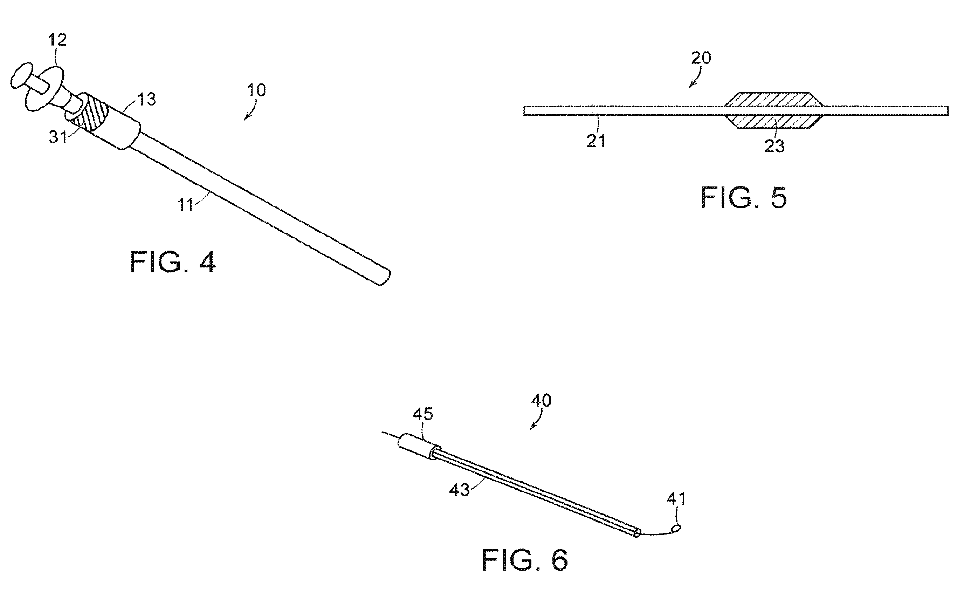

FIG. 4 is a perspective view of an illustrative delivery device according to one embodiment of the invention. According to the illustrative embodiment, the delivery device 10 includes a penetration device 11 (*e.g.*, a needle or a catheter) in fluid communication with a dispensing container 13, loaded with the cells or cell-containing flowable composition 31. According to one embodiment, the delivery device further includes a control module 12, for example, a syringe.

FIG. 5 is a perspective view of an illustrative marker device according to one embodiment of the invention. According to the illustrative embodiment, the delivery device (not shown) is used in conjunction with a marker device 20 which is inserted, for example, endoluminally or percutaneously, to mark the site of administration. According to one embodiment, the marker device 20 is a balloon catheter 21 having an inflatable balloon portion 23 including a detectable label, for example a fluoroscopically detectable label, to identify the target site of administration. According to another embodiment (not shown), the marker device 20 is a stent, for example, an at least partially radio-opaque vascular stent. According to another embodiment (not shown), the marker device 20 is an intraluminal imaging system, for example endoscopy. According to a further embodiment (not shown), the marker device 20 is a contrast agent or other agent visible under ultrasound, fluoroscopy, or other imaging system added to the cells of the flowable composition loaded within the penetrating device to permit imaging of and determination of the position of the penetrating device for placement of the cells or cell-containing flowable composition within a patient's body using, for example, contrast angiography, fluoroscopy or ultrasound. According to another embodiment, naturally occurring anatomical landmarks can be used as markers.

FIG. 6 is a perspective view of an illustrative guidance system delivery device according to one illustrative embodiment of the invention. According to the illustrative embodiment, the delivery device (not shown) is used in conjunction with a guidance system 40, such as a guidewire 41, which is inserted percutaneously to subsequently guide the delivery device to the site of administration. The guidance system 40 can have a diameter smaller than that of the delivery device 10 to minimize tissue damage caused by exploration for the desired site of administration. Optionally, the guidewire 41 is inserted using a guidewire delivery catheter 40 including, for example, a penetration device 43, such as a needle or catheter, and a handle 45 for manipulation of the guidance system delivery device by the operator.

### Dispensing Container

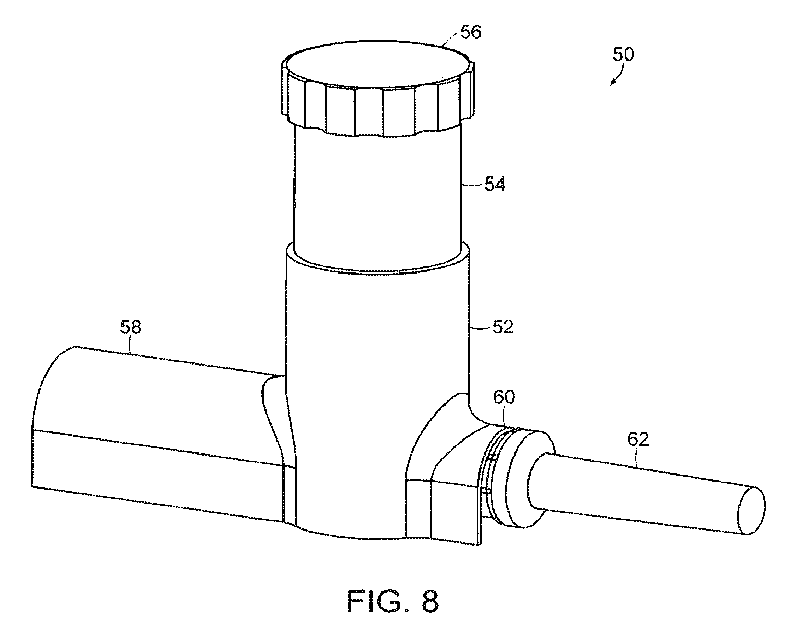

Figure 8 is a perspective view of an exemplary dispensing container 50, according to one illustrative embodiment of the invention. According to one embodiment, the dispensing container 50 includes a central material chamber section 52, a strainer column section 54 disposed above the central material chamber section 52, a cap 56 associated with the strainer column section 54, a rinse solution chamber section 58 associated with the central material chamber section 52, and a syringe engagement section 60. The syringe engagement section 60 can reversibly associate with a syringe 62, for example.

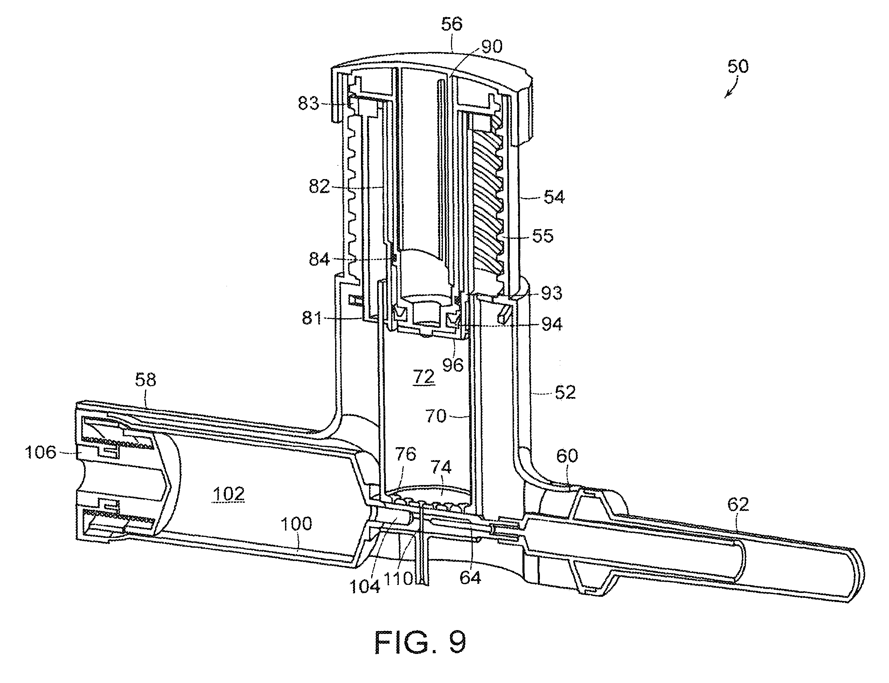

Figure 9 is a cut-away view of the illustrative dispensing container 50 of Figure 8 according to one embodiment of the present invention. According to one embodiment, the dispensing container 50 includes a central material chamber section 52. The central material chamber section 52 includes a material chamber 70 defining a material reservoir 72. The material chamber 70 further includes a base 74 including holes 76 associated, serially, with port 110, rinse port 104 and syringe port 64.

Referring to Figure 9, the dispensing container 50 further includes a strainer column section 54. The strainer column section 54 includes an outer cylindrical member 82. According to one embodiment, the strainer column section 54 includes two cylindrical members, one disposed within the lumen of the other, oriented substantially about the same longitudinal axis. According to one embodiment, the outer cylindrical member 82 is disposed within the material reservoir 72 of the material chamber 70, while the inner cylindrical member 93 is disposed within the lumen of the outer cylindrical member 82. According to one embodiment, the inner cylindrical member 93 includes a sealing member 81 disposed, for example, on at least one of the outer cylindrical member 82 and/or the material reservoir 72 to create a substantially liquid-tight seal between the material reservoir 72 and the outer cylindrical member 82.

Still referring to Figure 9, the strainer column section 54, according to one embodiment, is substantially cylindrical and includes at least one graduated sloped thread 55 within the inner wall of the strainer column section 54. The thread 55 is adapted to engage an extension 83 associated with the inner cylindrical member 93. According to one embodiment, the extension 83 is disposed within the thread 55 and, when the cap 56 turns, opening a valve created by an obliquely oriented o-ring 84 and an oblique diametrical reduction in outer cylindrical member 82, and releasing the outer cylindrical member 82 from a locked position, the extension 83 then rides within the thread 55 and descends along the thread 55 to lower the outer cylinder member 82 and inner cylindrical member 93 within the strainer column section 54 and towards the central material chamber section 52.

With continued reference to Figure 9, the strainer column section 54 further includes a straining screen 96 mounted on a outer cylindrical member 82. The straining screen 96 moves with the outer cylindrical member 82 as it descends in the central material chamber section 52.

The inner cylindrical member 93 is associated with and aligned with a cap column 90. The cap column 90 is attached to the cap 56 and orients the inner cylindrical member 93 and the outer cylindrical member 82 along the vertical axis of the dispensing container 50. The inner cylindrical member 93 and the cap column 90 define a media reservoir 92. The inner cylindrical member 93 further includes a cup seal 94 and an oblique ring seal 84, each disposed between the inner cylindrical member 93 and the outer cylindrical member 82 to create a substantially liquid-tight seal between the material reservoir 72 and the strainer column 54. The ring seal 84, according to one embodiment, is oriented in a sloped configuration so that, upon rotation of the cap 56 and release of the outer cylindrical member 82, the ring seal 84 is displaced from its sealing position and allows fluid, for example, transport media or rinse solution, to flow from the material reservoir 72, past the ring seal 84, and into the media reservoir 92.

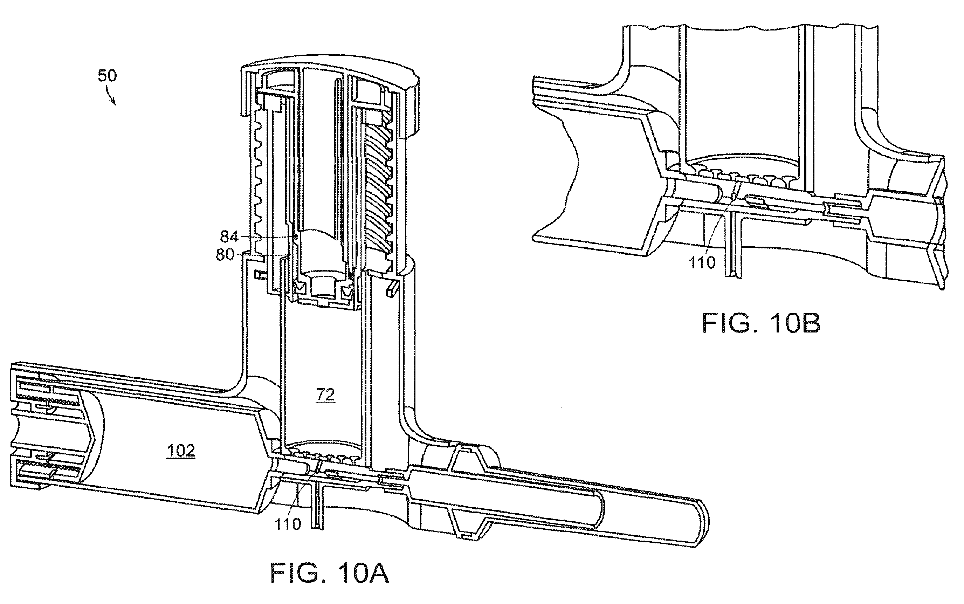

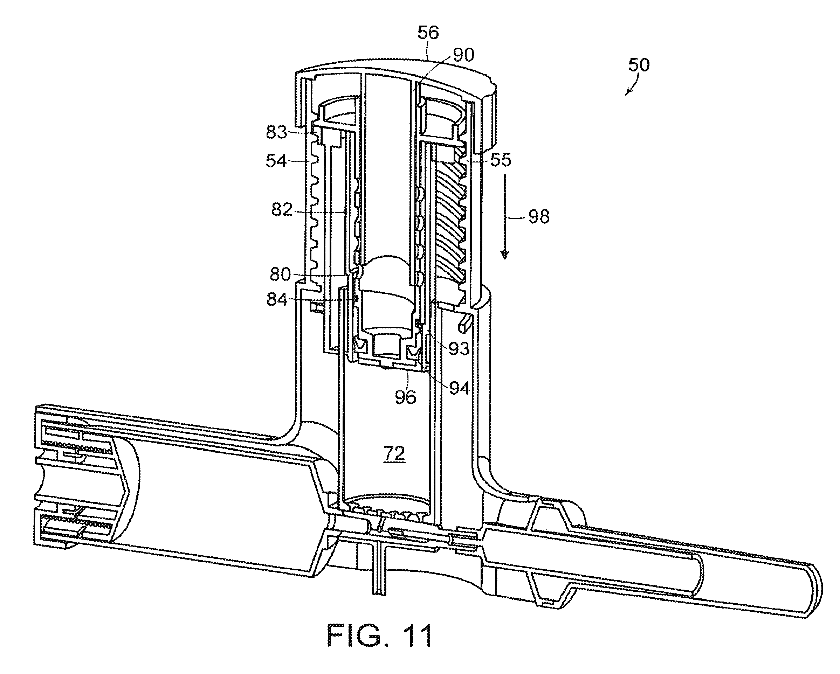

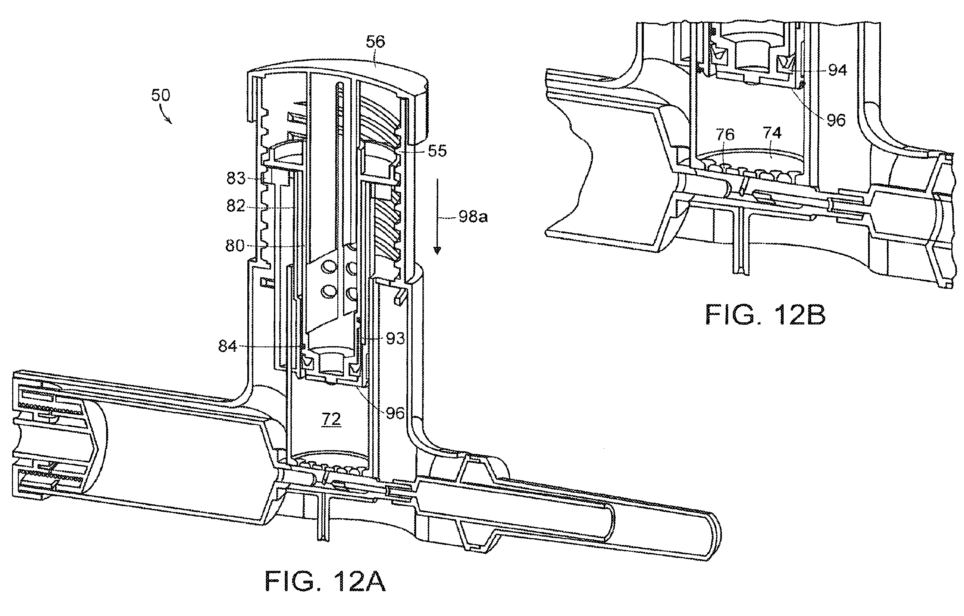

The obliquely oriented o-ring 84 is disposed, according to one embodiment, at an angle, for example, a 45° angle relative to the horizontal plane of the dispensing container. With reference to Figure 10A, when in the closed position, the o-ring 84 is positioned within a step 80 positioned in the outer cylindrical chamber 82. According to one embodiment, the step 80 is disposed at an angle relative to the horizontal plane of the dispensing container, for example, a 45° angle or an angle equivalent to the angle of the o-ring 84. When the o-ring 84 is positioned in a plane substantially parallel to the plane of the step 80, the o-ring 84 valve is in a sealed position. The o-ring 84 valve can open by rotation of the cap 56. Referring to Figure 11, when the cap 56 is rotated, the o-ring 84 twists along the vertical axis of the inner cylindrical member 93. As depicted in Figure 11, twisting the o-ring 84 out of alignment with the step 80 results in the introduction of a partial opening between the material reservoir 72 and the media reservoir 92. Referring to Figure 12A, additional twisting of the o-ring 84 out of alignment with the step 80 results in the introduction of a full opening between the material reservoir 72 and the media reservoir 92. However, when the o-ring 84 valve opens, there is no volume change in the material reservoir 72. Accordingly, opening the o-ring 84 valve does not compress the fluid or create a vacuum within the material reservoir 72.

With further reference to Figure 9, the dispensing container 50 further includes a rinse solution chamber section 58. According to one embodiment, the rinse solution chamber section 58 includes a rinse chamber 100 defining rinse reservoir 102. The rinse chamber 100 includes a rinse plunger 106 disposed within the rinse chamber 100 and forming a liquid-tight seal between the rinse chamber 100 and the rinse plunger 106 to prevent the release of rinse solution from the rinse reservoir 102. The rinse reservoir 102 is, optionally, when the rinse port 104 is in the open position, in fluid communication with the material reservoir 72 through the rinse port 104. Further, the material reservoir 72 is, optionally, when the syringe port 64 is in the open position, in fluid communication with the syringe port 64. The syringe port 64 can be associated with a syringe 62, for example. Finally, the material reservoir 72 is, optionally, when the port 110 is in the open position, in fluid communication with port 110. According to one embodiment, port 110 includes a needle puncturable diaphragm (not shown) adapted to allow introduction of flowable composition into the material reservoir 72, for example, prior to storage and/or shipping. Following introduction of flowable composition into the material reservoir 72, the diaphragm closes to maintain sterility of material reservoir 72.

Figures 10A and 10B are cut-away views of the illustrative dispensing container 50 of Figure 8 according to one embodiment of the present invention. According to one embodiment, as depicted in Figures 10A and 10B, the dispensing container is configured for material transport and storage. In this configuration, the material reservoir 72 is filled with material, for example, flowable composition. According to one embodiment, the material is in the material reservoir 72 with a sufficient quantity of media, for example, transport media, to maintain the material in a viable and functional state, as described above. In this configuration, the rinse reservoir 102 is filled with rinse solution, for example, saline solution. Further, in this configuration, the port 110, rinse port 104 and syringe port 64 are all in a closed position to maintain the material is a closed system and to prevent the material from releasing during transport and/or storage.

Figure 11 is a cut-away view of the illustrative dispensing container 50 of Figure 8 according to one embodiment of the present invention. According to one embodiment, to extract the media or other substantially liquid solution from the material, the cap 56 is rotated, for example, clockwise, to displace the ring seal 84 from its sealed position to its open position, to release the outer cylindrical member 82, and to engage the extension 83 with the thread 55. Once the extension 83 is engaged with the thread 55 and the extension begins moving downward along the sloped thread 55, the outer cylindrical member 82 and inner cylindrical member 93 begin moving downward in the direction indicated by arrow 98. When the outer cylindrical member 82 and the inner cylindrical member 93 travel downward, fluid is able to pass through the straining screen 96 from the material reservoir 72 to the media reservoir 92. The cup seal 94 acts as a check valve to prevent the upward movement of the straining screen 96 and, in conjunction, to prevent the possibility of extracted fluid circulating back into the material reservoir 72 at some point after the fluid is removed from the material reservoir 72. During the fluid extraction process, the material, which consists substantially of cells and/or particles having a diameter larger than the diameter of the screen perforations of the straining screen 96, remains within the material reservoir 72.

Figures 12A and 12B are cut-away views of the illustrative dispensing container 50 of Figure 8 according to one embodiment of the present invention. According to one embodiment, the outer cylindrical member 82 and the inner cylindrical member 93 continue moving in a downward direction, as indicated by arrow 98a, as the extension 83 moves along the thread 55. In conjunction, the fluid in the material reservoir 72 continues to move into the media reservoir 92 and the material remaining within the material reservoir 72 becomes more concentrated. With reference to Figure 12B, according to one embodiment, the straining screen 96 is disposed within the inner cylindrical member 93 at an angle, such that the screen 96 extends from the margin of the inner cylindrical member 93 to the center of the lumen of the inner cylindrical member 93 at an angle of, for example, about 15 degrees downward relative to the horizontal plane of the dispensing container 50. Similarly, according to one embodiment, the base 74 of the material chamber 70 is disposed at an angle, such that the base 74 extends from the margin of the material chamber 70 to the center of the material chamber 70 at an angle of, for example, about 15 degrees downward relative to the horizontal plane of the dispensing container 50.

With continued reference to Figure 12B, the base 74 further includes at least one hole 76 extending from the material reservoir 72 to the port 110, the rinse port 104 and/or the syringe port 64. According to one embodiment (not shown), the base 74 includes one hole 76 disposed at the center of the base 74. According to another embodiment, the base 74 includes a series of holes 76 disposed in a substantially linear pattern extending substantially across the base 74 and through the center of the base 74. According to a further embodiment (not shown), the base 74 includes a series of holes 76 disposed in a substantially circular pattern across the base 74. According to one embodiment, the holes 76 arranged in a substantially circular pattern across the base 74 are positioned close to the margin of the material chamber 70 to facilitate turbulence and material mixing during the rinse procedure, described in greater detail below.

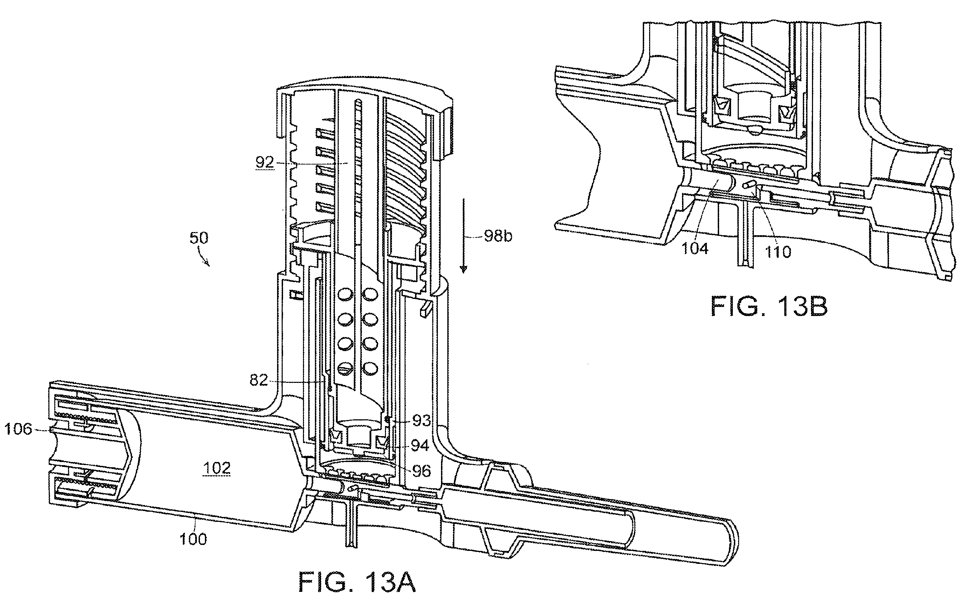

Figures 13A and 13B are cut-away views of the illustrative dispensing container 50 of Figure 8 according to one embodiment of the present invention. According to this embodiment, the outer cylindrical member 82 and inner cylindrical member 93 continue moving in the downward direction indicated by arrow 98b until the outer cylindrical member 82 engages with a detent (not shown) on the rinse chamber 100 and can no longer move downward. In this position, a significant portion of the fluid originally associated with the material in the material reservoir 72 has been separated from the material and now resides in the media reservoir 92. According to one embodiment, the outer cylindrical member 82 engages the detent (not shown) on the rinse chamber 100 to rotate the rinse chamber 100, thereby opening rinse port 104 so that the rinse reservoir 102 is in fluid communication with the material reservoir 72.

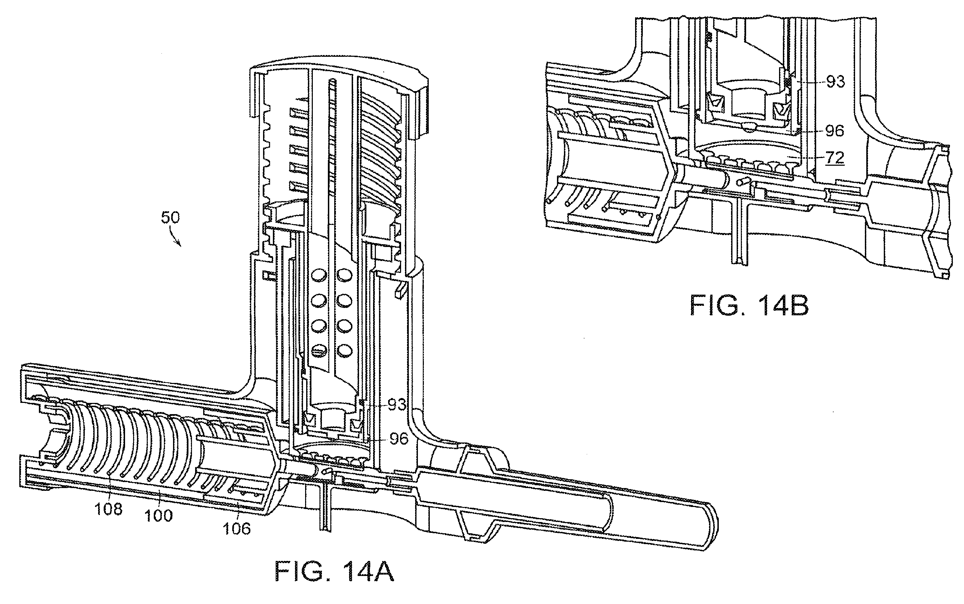

With continued reference to Figures 13A and 14B, once the rinse port 104 is open, the rinse plunger 106 can be activated to advance rinse solution contained within the rinse reservoir 102 through the rinse port 104 and into the material chamber 72. Once the inner cylindrical member 93 is in the lowered position, the engagement 83 and thread 55 prevents the inner cylindrical member 93 from moving upward to expand the volume of the material reservoir 72. According to one embodiment, the straining screen 96 is positioned approximately 5 mm above the base 74, creating a material reservoir 72 volume of approximately 9.5 cc. Accordingly, as the rinse solution advances into the material chamber 72, it can either displace remaining media within the material and push the remaining media into the media reservoir 92 or rinse the material and pass through the straining screen 96 into the media reservoir 92. Accordingly, during the rinse procedure, the rinse solution passes through the material contained within the material reservoir 72 and then advances past the straining screen 96 into the media reservoir 92, carrying remaining media and other material contaminants out of the material. During the rinse procedure, the volume of the material reservoir 72 remains constant.

Figures 14A and 14B are cut-away views of the illustrative dispensing container 50 of Figure 8 according to one embodiment of the present invention. According to one embodiment, the rinse plunger 106 advances by the force of the spring 108 toward the rinse port 104 until substantially all of the rinse solution passes from the rinse reservoir 102 through the material reservoir 72 and into the media reservoir 92.

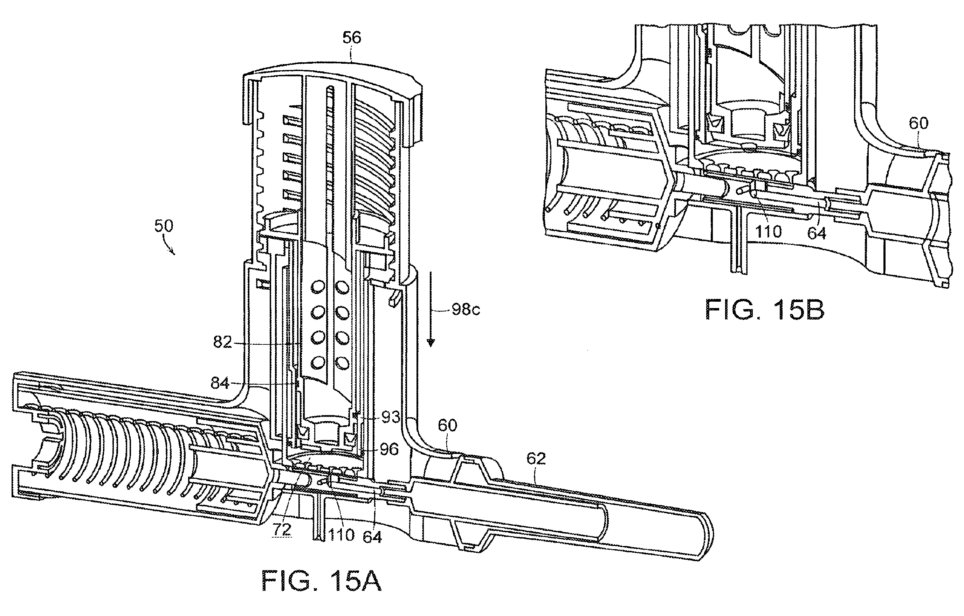

Figures 15A and 15B are cut-away views of the illustrative dispensing container 50 of Figure 8 according to one embodiment of the present invention. According to one embodiment, following completion of the rinse procedure and prior to initiating material transfer to the syringe 62, the rinse chamber 100 rotates to remove the detent (not shown) and to allow the outer cylindrical member 82 to continue its descent. The cap 56 twists to re-engage the ring seal 84 in its closed position, thereby preventing fluid from flowing from the material reservoir 72 into the media reservoir 92. The syringe port 64 rotates or otherwise engages to open the syringe port 64 so that the syringe 62 is in fluid communication with the material reservoir 72. The outer cylindrical member 82 continues movement in the downward direction indicated by arrow 98c, pushing the material out of material chamber 72, through holes 76 in base 74 and through syringe port 64, into syringe 62.

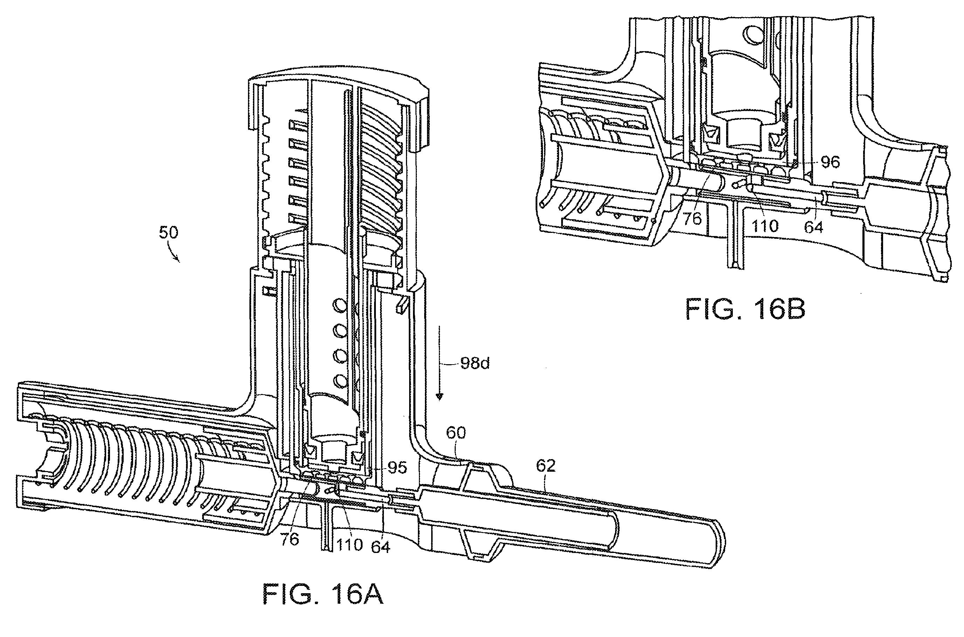

Figures 16A and 16B are cut-away views of the illustrative dispensing container 50 of Figure 8 according to one embodiment of the present invention. According to one embodiment, the outer cylindrical member 82 continues moving in the downward direction indicated by arrow 98d until the straining screen 96 of the inner cylindrical member 93 is in contact with the base 74 of the material chamber 70. Completion of this first material transfer push results in approximately 5 cc of material transferring from the material reservoir 72 to the syringe 62. Following completion of the first material transfer push, cap 56 is rotated to advance an additional approximately 6-7 cc of rinse solution from between the inner cylindrical member 93 and the straining screen 96, through the straining screen 96, and into the syringe 62. This second material transfer push results in an additional approximately 4.5 cc of material transferring to the syringe 62, cleansing any residual material from the material reservoir 72. Upon completion of the second material transfer push, the syringe contains approximately 9.5 cc of material, of which approximately 3.5 cc comprises flowable composition and the remaining 6 cc comprising rinse solution. The remaining rinse solution is extracted from the flowable composition in the syringe 62 prior to delivery, as described in greater detail below.

### Delivery Springe

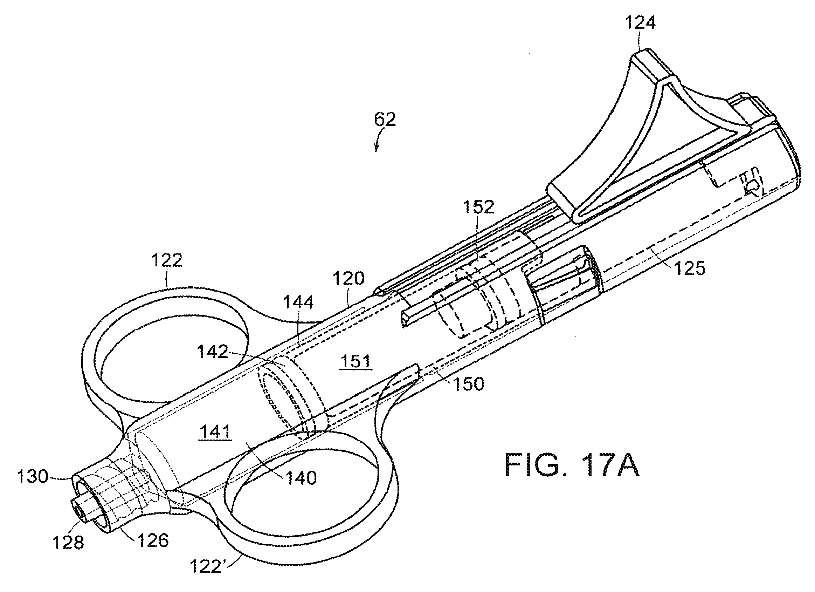

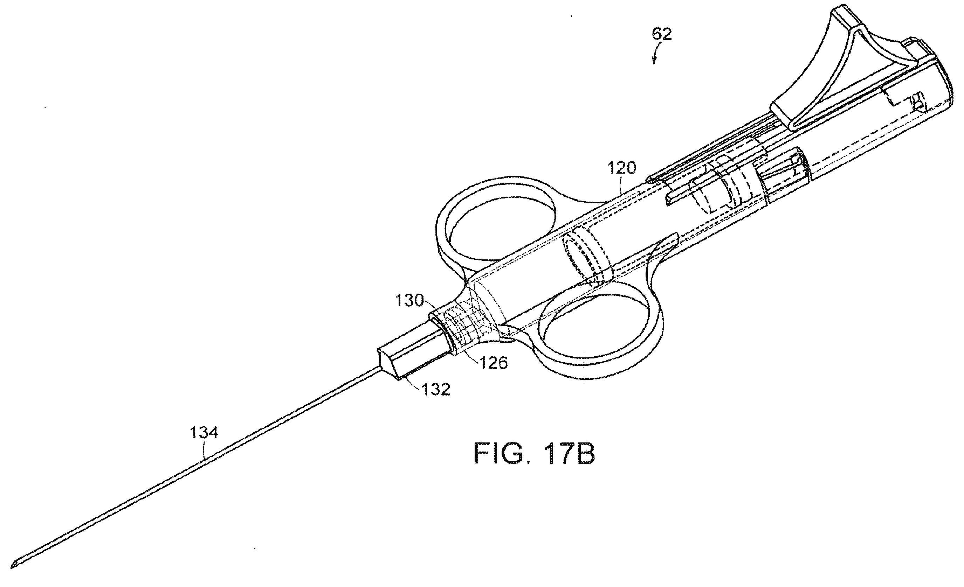

Figure 17A is a perspective view of an illustrative delivery syringe 62 according to one embodiment of the present invention. According to one embodiment, the delivery syringe 62 includes a syringe housing 120 including one or more, for example, two, handles 122 and 122' and a lever 124. The syringe housing 120, according to one embodiment, can be substantially cylindrical and define a lumen 141. According to one embodiment, the syringe housing 120 further includes two plungers, an outer plunger 150 and an inner plunger 125, each disposed within the syringe housing 120 and slidingly moveable within the syringe lumen 141. According to one embodiment, the inner plunger 125 is at least partially disposed within the lumen 151 of the outer plunger 150. According to one embodiment, the outer plunger 150 contains an outer ring seal 142, for example, disposed at the distal end of the outer plunger 150, for example, at the end of the outer plunger 150 closest to the needle housing 126. According to one embodiment, the outer plunger 150 further contains a plunger screen 144 disposed at the distal end of the outer plunger 150, for example, at the end closest to the needle housing 126. According to one embodiment, the inner plunger 125 contains an inner ring seal 152, for example, disposed at the distal end of the inner plunger 125, for example, at the end of the inner plunger 125 closest to the needle housing 126. According to one embodiment, the inner ring seal and/or the outer ring seal provide a liquid-tight seal between the syringe lumen 141 and the plunger lumen 151 to prevent the flow of material between the two lumens. However, the plunger screen 144 of the outer plunger 150 allows the passage of fluid from the syringe lumen 141 into the plunger lumen 151 during the material loading procedure, described in greater detail below. The syringe housing 120 further includes a needle housing 126 containing a threaded bore 130, Luer lock, or other engagement mechanism to engage a puncture device, for example, a needle (shown in Figure 17B), catheter or tube. The needle housing 126 further contains a needle port 128 for introduction and expulsion of material from the syringe 62.

Figure 17B is a perspective view of an illustrative delivery syringe 62 and needle 134 according to one embodiment of the present invention. According to one embodiment, the delivery syringe 62 includes a syringe housing 120 and further includes a needle 134 associated with the needle housing 126. According to one embodiment, the needle 134 includes a needle attachment mechanism 132, for example, a threaded bore, Luer lock, or other engagement mechanism to engage the needle housing 126 and, preferably, matched to the engagement mechanism of the syringe 62.

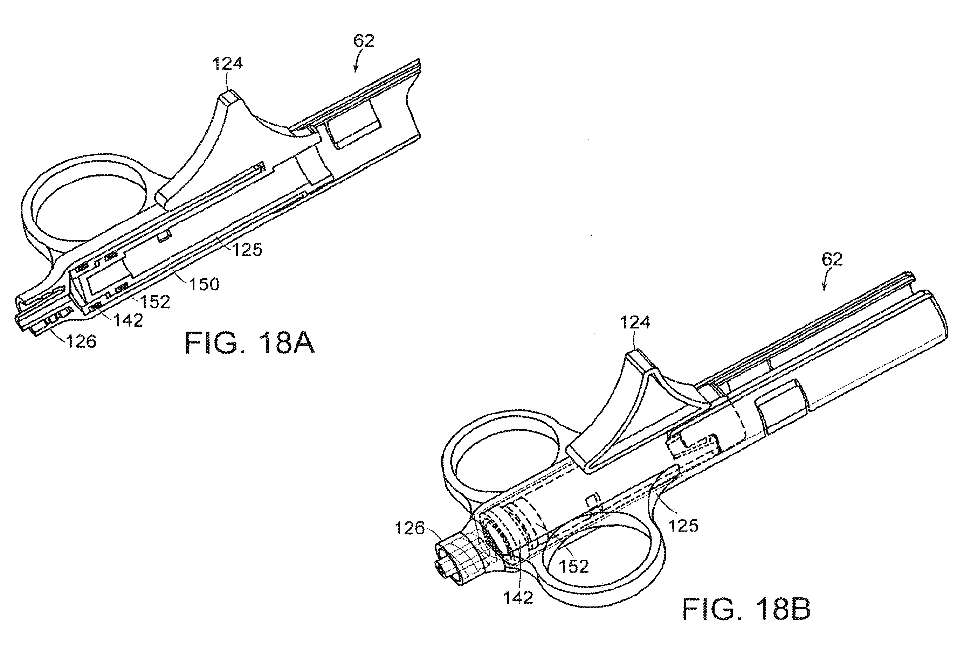

Figure 18A is a cut-away view and Figure 18B is a perspective view of the illustrative delivery syringe 62 of Figure 17A according to one embodiment of the present invention. According to one embodiment, prior to introduction of the material into the syringe 62, the lever 124 is in its most distal position, the distal end of the inner plunger 125 is compressed against the distal end of the luminal surface of the outer plunger 150 and the distal end of the outer plunger 150 is compressed against the distal end of the luminal surface of the syringe lumen 141. According to this embodiment, the syringe lumen 141 contains a minimum volume.

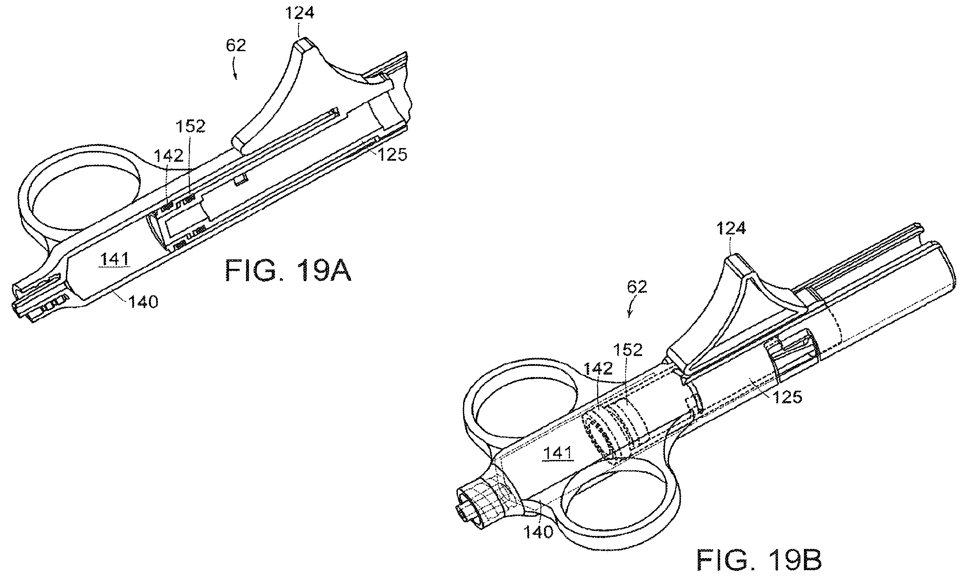

Figure 19A is a cut-away view and Figure 19B is a perspective view of the illustrative delivery syringe 62 of Figure 17A according to one embodiment of the present invention. According to one embodiment, during the first stage of material introduction into the delivery syringe 62, the lever 124 is retracted proximally, away from the needle housing 126. During this first stage of retraction, the outer plunger 150 and the inner plunger 125 retract together, with the distal end of the inner plunger 125 remaining adjacent to the distal end of the luminal surface of the outer plunger 150. Retraction of the outer plunger 150 creates a material chamber in the syringe lumen 141, defined by the syringe luminal space between the needle housing 126 and the distal end of the outer plunger 150, which according to one embodiment includes the outer ring seal 142. During the first stage of material introduction, the material is introduced into the material chamber 141 as the plungers 150 and 125 are retracted.

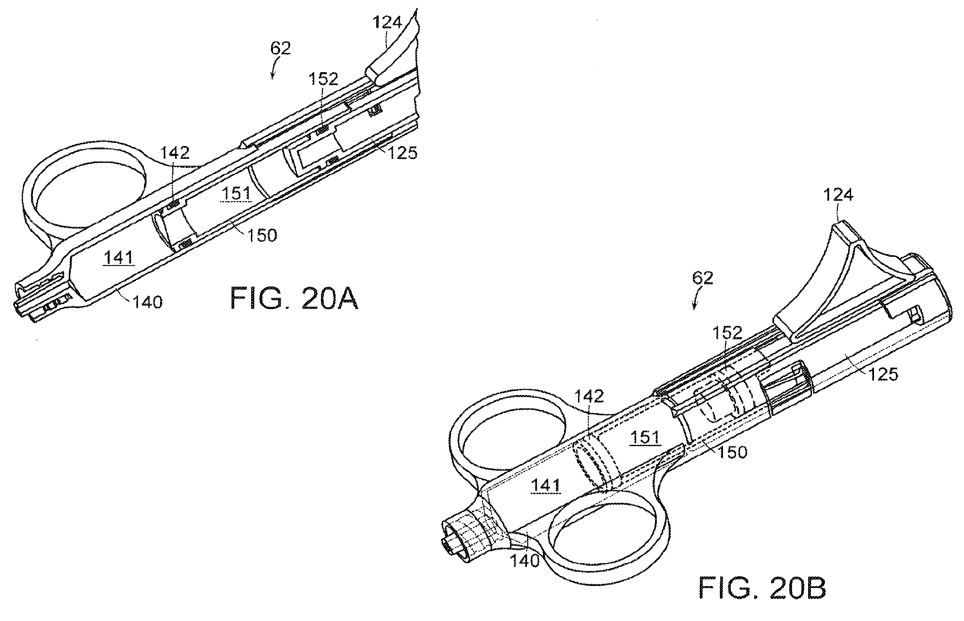

Figure 20A is a cut-away view and Figure 20B is a perspective view of the illustrative delivery syringe 62 of Figure 17A according to one embodiment of the present invention. According to one embodiment, during the second stage of material introduction into the delivery syringe 62, the lever 124 is further retracted proximally, away from the needle housing 126. During this second stage of retraction, according to one embodiment, the outer plunger 150 remains substantially stationary and the inner plunger 125 is further retracted proximally. Retraction of the inner plunger 125 creates an excess fluid collection chamber in the plunger lumen 151, defined by the outer plunger luminal space between the distal end of the outer plunger 150 and the distal end of the inner plunger 125, which according to one embodiment includes the inner ring seal 152. During the second stage of material introduction, additional material is introduced into the material chamber 141 and fluid, for example, rinse solution, is introduced into the excess fluid collection chamber 151. Because the plunger screen 144 prevents the transfer of material, for example, the flowable composition, from the material chamber 141 into the excess fluid collection chamber 151, the second stage of material introduction includes drawing additional rinse solution through the material contained within the material chamber 141 and into the excess fluid collection chamber 151. Further, because the second stage of material introduction also includes drawing additional material into the material chamber 141, the material in the material chamber 141 becomes more concentrated during the second stage of material introduction.

During the second stage of retraction, according to an alternative embodiment (not shown), the outer plunger and the inner plunger are further retracted proximally together. According to this embodiment, the additional proximal retraction of the outer plunger and the inner plunger enlarges the volume of the material chamber. Following further retraction of both the outer plunger and the inner plunger, the outer plunger, including the plunger screen, is advanced in a distal direction, toward the needle housing. Advancement of the outer plunger and the plunger screen results in reduction of the volume of the material chamber, movement of fluid from the material chamber, through the plunger screen to the excess fluid collection chamber, and, accordingly, concentration of the flowable composition remaining within the material chamber.

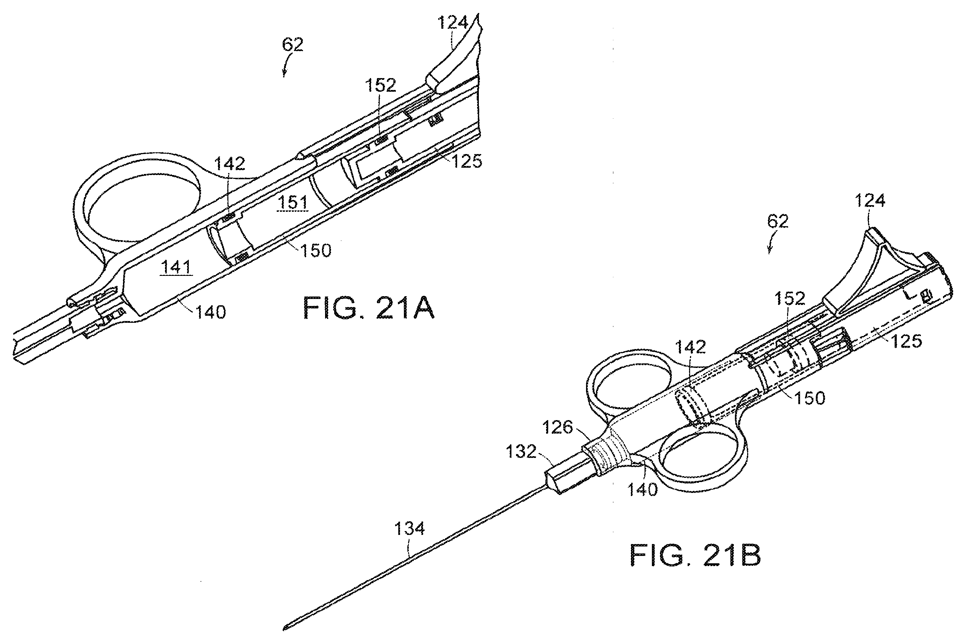

Figure 21A is a cut-away view of the illustrative delivery syringe 62 of Figure 17A and Figure 21B is a perspective view of the illustrative delivery syringe 62 with needle 134 of Figure 17B according to one embodiment of the present invention. According to one embodiment, during the delivery stage, the outer plunger 150 and the inner plunger 125 are locked in position relative to one another, such that distal movement of the lever 124 results in coordinated movement of the inner plunger 125 and the outer plunger 150 toward the needle housing 126 without reducing the volume of the excess fluid collection chamber 151 or, as a result, expelling more than an insubstantial amount of rinse solution from the excess fluid collection chamber 151 into the material chamber 141. During the delivery stage, the material is compressed in the material chamber 141 by the force of the plunger screen 144 on the outer plunger 150, and expelled from the material chamber 141, through the needle port 128 and through the lumen of the needle 134 to the desired site of administration. An advantage of the present invention is that the double lumen design allows transfer of a thinner, more flowable material during loading of the material into the delivery syringe 62, while only expelling a more concentrated material and removing the rinse solution from the material into the excess fluid collection chamber 151 to prevent delivery of more than an insubstantial amount of the rinse solution to the patient during delivery.

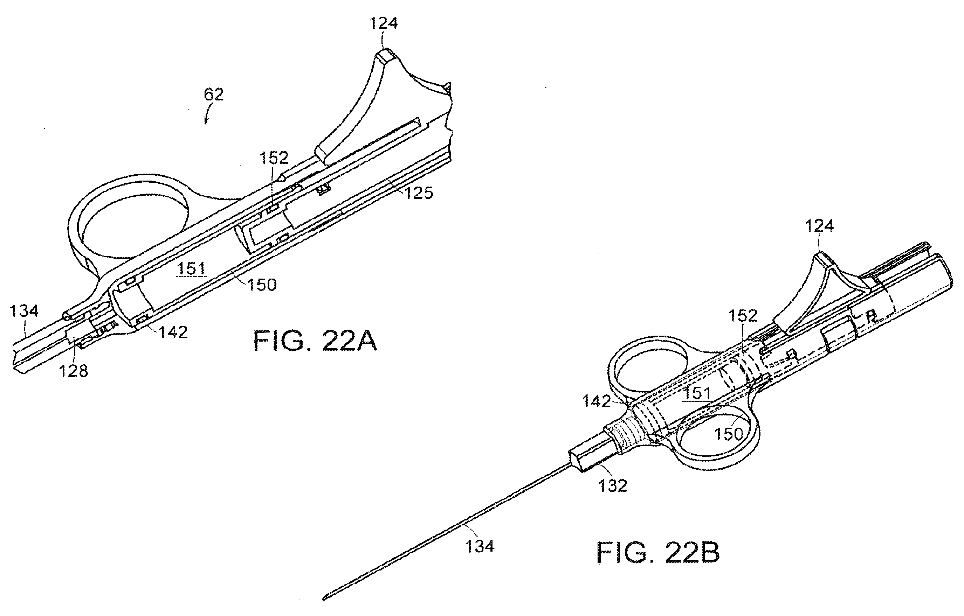

Figure 22A is a cut-away view of the illustrative delivery syringe 62 of Figure 17A and Figure 22B is a perspective view of the illustrative delivery syringe 62 with needle 134 of Figure 17B according to one embodiment of the present invention. According to one embodiment, at the completion of the delivery stage, the plunger screen 144 of the outer plunger 150 is advanced distally toward the needle housing 126 and, according to one embodiment, adjacent the distal end of the luminal space of the material chamber 141.

### Methods of Minimally-Invasive Delivery

The present invention finds application in a method for delivering or administering cells or a cell-containing flowable composition to a non-luminal site of administration within a subject. For purposes of the present invention generally, a site of administration includes locations at, into, adjacent to, upstream of, downstream of, or in the vicinity of a site of damage, disease or coincident interventional treatment of the structure. Further, it is contemplated that a non-luminal site, also termed an extraluminal site, can include an exterior surface or a non-luminal component of a tubular structure, or an interior surface or an exterior surface or volume of a soft tissue structure or an organ. In the case of tubular vascular structures, non-luminal surfaces include, for example, sites on or within the adventitia, media, or intima of the tubular vascular structure, sites on the interior and/or exterior surface or volume of the vascular sheath and/or muscular sheath, or on the exterior surface and/or within the adjacent muscle tissue. For purposes of this invention, non-luminal or extraluminal is any component of a tubular or solid tissue or organ structure except the interior surface of the lumen of the tubular structure.

In such applications, the cells or the cell-containing flowable composition can be administered in a variety of configurations at the site of administration, For example, the cells or the cell-containing flowable composition can be administered in a linear application, for example, parallel to the longitudinal axis of the tubular structure; in a circumferential application, for example, perpendicular to the longitudinal axis of the tubular structure; or in a mass at the site of administration. An adjacent site may be within about 0 mm to 40 mm of the site of an injured or diseased target site, or within about 2 mm to 20 mm of the site of an injured or diseased target site. Alternatively, an adjacent site is any other clinician-determined adjacent location where the deposited cells or the cell-containing flowable composition is capable of exhibiting a desired effect. In some clinical subjects, insertion of the penetrating device at an injured or diseased target site could disrupt the injured or diseased target site resulting in further adverse clinical sequelae. Accordingly, in such subjects, care should be taken to insert the penetrating device at a location a distance from an injured or diseased target site, preferably a distance determined by the clinician governed by the specific circumstances at hand.

According to one application of the invention, the cells or the cell-containing flowable composition is delivered locally to a surgically-exposed site at, adjacent to, and/or in the vicinity of a target site in need of treatment. In this case, delivery is guided and directed to the desired target site of administration by direct observation. Delivery can further be aided by the optional, coincident use of an identifying step as described below.

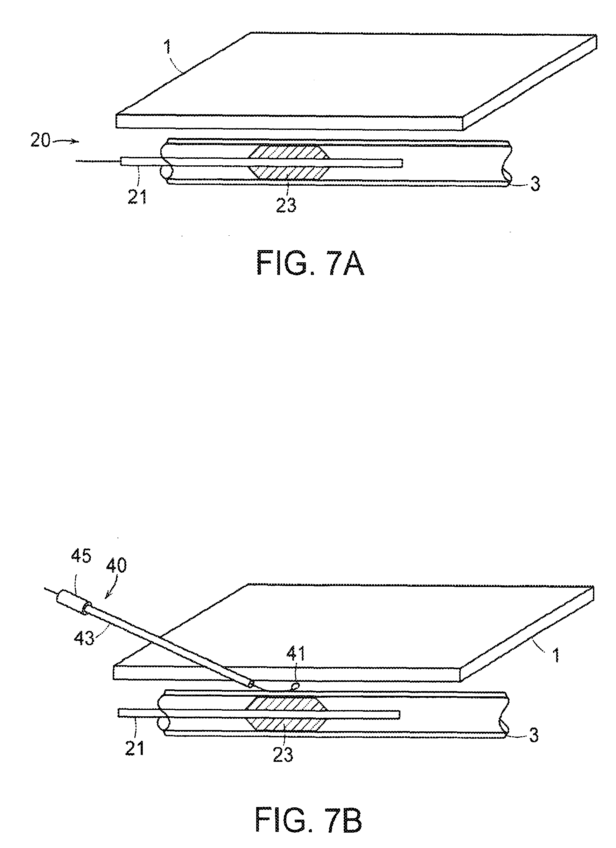

In other applications, the cells or the cell-containing flowable composition is delivered percutaneouly or extraluminally using a suitable delivery device, optionally in conjunction with a suitable marker device and/or guidance device, the features of which are described in greater detail above. FIGS. 7A-7D depict a series of steps according to one illustrative method of percutaneous or extraluminal delivery. FIG. 7A depicts a layer of skin 1 or other tissue overlying a tubular structure 3, such as a blood vessel. According to this method, the tubular structure 3 depicted in FIG. 7A contains a site in need of treatment. A marker system delivery device 20, for example, a balloon catheter 21, is inserted endoluminally to locate and mark a desired site of administration. The balloon portion 23 of the balloon catheter 21 may further include a detectable label or marker (not shown) which allows an operator to determine the location of the balloon portion 23 of the marker device within the subject and thereby to identify the desired target site of administration. Additional exemplary marker systems (not shown) include a detectable stent, balloon or other mechanical marker. Additionally, certain anatomical structures, including solid organs and tumors, are visible and distinguishable from the surrounding tissue and can serve as markers during location of the site of administration.

With reference to FIG. 7B, a guidance system delivery device 40, for example, a guidewire 41 inserted using a guidewire catheter 43, is inserted percutaneously through the layer of tissue 1 to the desired site of administration. The guidance system can be used with or without the marker system, for example, the balloon portion 23 of the balloon catheter 21. The guidance system delivery device 40 may include a needle 43 for penetrating the tissue layer 1 and a handle 45 to facilitate manipulation of the guidance system delivery device 40 by the operator. Guidewire 41 is disposed within the lumen of the needle 43 prior to and during insertion of the guidance system delivery device 40 to the site of administration. Once the guidewire 41 is in place, for example, with the distal end of the guidewire at the intended site of administration, the guidance system delivery device 40 is removed from the subject, leaving the guidewire 41 in place. Additional guidance devices include, but are not limited to, stents, staples, other radioopaque or otherwise visible devices or portions of devices previously or contemporaneously implanted in the patient and visible using a guidance system.

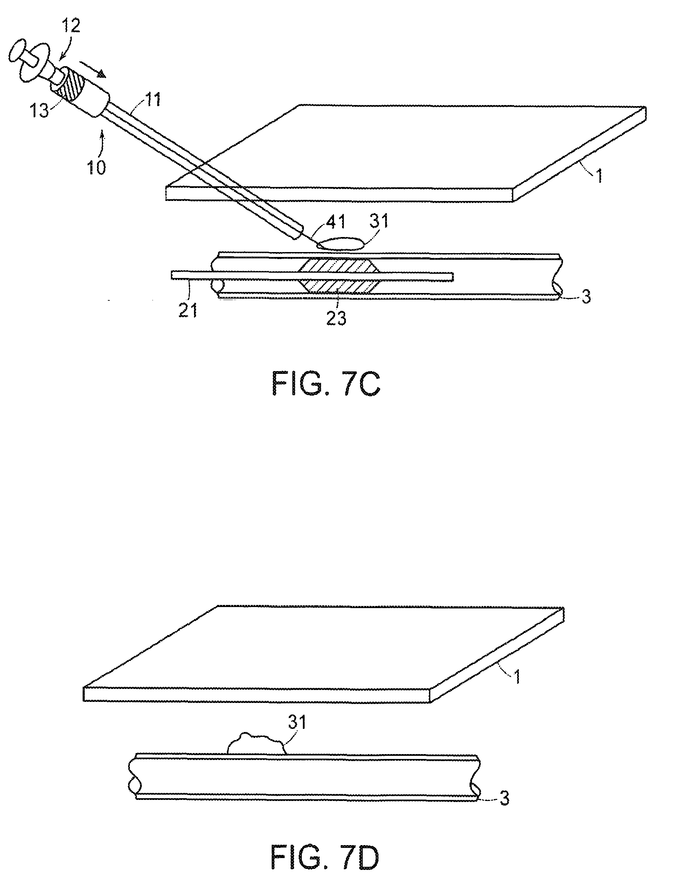

With reference to FIG. 7C, the delivery device 10 is inserted along the guidewire 41 to the desired site of administration. The control module 12 is manipulated by the operator to administer a desired volume of the cells or the cell-containing flowable composition 31 at the target site of administration, and optionally within the tissue pocket 51.

With reference to FIG. 7D, following administration of the cells or the cell-containing flowable composition 31 to the desired site of administration, the delivery device 10 (not shown) and guidewire 41 (not shown) are removed from the subject.

The flowable composition can be administered percutaneously coincident with ultrasound imaging guidance. According to this method, the ultrasound imaging system, for example, a medium to high frequency (approximately 5-12 MHz) linear array transducer, is placed at or near the intended site of administration and the intended site of administration located using, for example, 2-D and/or color-Doppler ultrasound. In the case of administration to a luminal structure, the ultrasound image can include a longitudinal view of the length of the luminal structure to be treated or a cross-sectional view of the circumference of the luminal structure to be treated.

Once the site of administration is located, the penetration device of the delivery device, preloaded with a dose of an appropriate flowable composition, is inserted through the overlaying layers of skin and other tissue and toward the site of administration. The penetration device may be inserted into the overlaying muscle layer adjacent the desired site of administration, between the adjacent muscle layer and the muscular sheath, between the muscular sheath and the vascular sheath, between the vascular sheath and the vessel, or into the adventitia, media or intima of the vessel. The placement of the penetration device at the desired site of administration may be confirmed using a guidance device, for example, an ultrasound imaging system in combination with an echogenic needle.

The penetration device may include a beveled tip. The angle of the beveled tip may be selected to create a bevel at or near the angle of approach, measured parallel to the surface of the structure to be treated, of the needle during insertion. The penetration device may be inserted at an angle and with a rotational orientation chosen to optimize the control of and the piercing function of the beveled tip of the penetration device.

The beveled tip of the penetration device may be inserted with a rotational orientation selected to position the ground surface of the bevel end of the beveled tip substantially parallel to the longitudinal axis of the structure to the treated. Orientation of the bevel tip can be adjusted according to the location of the structure to be treated and the physician selected route of approach to the structure to be treated to optimize the control of and the piercing function of the beveled tip of the penetration device.

The penetration device may be inserted into the skin adjacent the end of the ultrasound transducer contact and in the plane of the ultrasound imaging pattern. With the penetration device aimed toward the target, and using the image of the penetration device under ultrasound, the tip of the penetration device is guided to the desired site of administration, for example, adjacent a blood vessel outer wall, but not puncturing the vessel.

The beveled tip of the penetration device may be inserted through the femoral sheath into the space adjacent the blood vessel. According to this and similar embodiments of delivery to sheathed structures, the operator end of the delivery device can be tilted downward and the beveled tip deflected upwards, resulting in a tenting or expansion of the femoral sheath and, in some situations, possible coordinated deflection of the adjacent blood vessel, thereby confirming the location of the beveled tip within the femoral sheath. If the sheath and/or the vessel do not tent or expand when the tip of the penetration device is deflected, the penetration device can be further advanced. According to some exemplary sheathed structures, a tactile release or pop can be sensed by the operator once the tip of the penetration device has penetrated the sheath. The placement of the beveled tip of the penetration device at the desired site of administration can be confirmed prior to administration of the implantable material using a guidance or imaging device, for example, ultrasound, fluoroscopy or angiography.

Once the tip of the penetration device is located at the desired site of administration, the flowable composition is admistered to the site. The flowable composition may be administered in a single dose, or in two or more sequentially administered doses, when the sequential doses can be administered at the same site or at adjacent sites. The flowable composition can be administered along a length of the structure adjacent to the site to be treated, around a portion of or around the entire circumference of the structure surrounding the site to be treated, or at a single site of deposition adjacent the site to be treated. A site of deposition may be about 2 mm to 40 mm long and adjacent to the site of an injured or diseased target site, or about 2 mm to 20 mm long and adjacent to the site of an injured or diseased target site.

Following deposition of the flowable composition at the site of administration, and prior to and/or following removal of the delivery device from the patient, the location of the flowable composition may be confirmed using a guidance device, for example, an ultrasound imaging system in combination with an echogenic material. The delivery device is then removed from the tissue and the patient. The delivery method can be repeated at the same site of administration, at an adjacent site of administration, or at multiple adjacent sites of adminstration, based on the site to be treated and the judgment of the administering physician.

The cells or the cell-containing flowable composition may be delivered endoluminally or endovascularly using a suitable catheter, catheter like or combination catheter-needle delivery device, optionally in conjunction with a suitable marker device, guidance device and/or dissection device, the features of which are described in greater detail above.

The delivery device may include an expandable balloon stabilizer to stabilize the movement and insertion of the needle at the site of administration, where the delivery device is prepared prior to introduction into the patient by preloading the expandable balloon stabilizer with saline. A syringe containing heparinized saline is attached to the delivery port of the catheter and the catheter is flush with heparinized saline, confirming passage through the entire length of the catheter and/or needle. Following confirmation, the saline syringe is removed from the delivery port. A second syringe is preloaded with a dose of the flowable composition. The syringe containing the flowable composition is attached to the delivery port of the catheter and a small amount of the flowable composition is flushed into the delivery lumen.

Following preparation of the catheter based delivery device, the femoral artery is accessed and the catheter tip inserted into the femoral artery. The delivery device may be inserted into and passed through a previously placed guiding catheter or sheath. The distal tip of the catheter is passed through the necessary and proper vascular passages under guidance, for example, fluoroscopic imaging and/or ultrasound imaging, until the catheter tip is located adjacent to the desired site of administration. Once located at the site of administration, the guidance transducer, for example, an ultrasound transducer, is aligned with the target vessel to obtain a longitudinal image of the vessel. According to one embodiment, the delivery device operator then initiates balloon inflation to stabilize the tip of the catheter within the vessel and to reduce the risk of inaccurate or multiple needle sticks at the site of administration. Following inflation of the balloon with saline, the balloon inflation port is closed.

Inflation of the stabilizing balloon may occur with coordinated extension of the needle into the vascular wall, or once the catheter is stabilized, the needle may be independently extended from within the catheter tip and toward the vascular wall. In either case, the needle is then inserted into the luminal surface of the vascular wall and into the vascular tissue. The needle may be extended into the vascular tissue until the tip of the needle is located within the intima, the media, the adventitia, the space exterior to the vessel but within the vascular sheath, exterior to the vascular sheath within the space adjacent the muscle or adjacent muscular sheath, or within the adjacent muscle tissue. Additionally, the needle can be configured to include a length extending from the catheter adapted for insertion into the desired tissue location. For example, if the desired site of administration is within the intima of the vessel, the needle may have a length exterior of the catheter body of, for example, 4 mm. Alternatively, if the desired site of administration is within the adjacent muscle tissue, the needle may have a length exterior of the catheter body of, for example, 10 mm. Alternative needle configurations are possible depending on the desired site of administration and the route of administration.

Once the tip of the needle is at the desired location for administration of the flowable composition, the syringe or other actuator on the handle or other operator interface of the delivery device is activated to administer the flowable composition. Once the dose of flowable composition is administered to the site of administration, the balloon inflation valve is opened, the balloon deflated and then the inflation valve closed. The delivery device is then removed from the patient. The location of the flowable composition may be confirmed using an imaging system, for example, ultrasound or fluoroscopy, when the location may be confirmed both longitudinally and transversely.

The implanted cells or cell-containing flowable composition can be visualized within the extraluminal space during or following administration using, for example, angiography, ultrasound imaging and/or spectral Doppler flow evaluation. Post-administration visualization may be conducted to ensure the cells or the cell-containing flowable composition has been delivered to the extraluminal space rather than to the luminal space and/or to ensure that the vessel wall was not inadvertently punctured.

It is also contemplated that the foregoing dissection and delivery steps can take place concurrently or sequentially. For example, the cells or the cell-containing flowable composition can itself be used to accomplish fluid dissection if delivered under pressure. Alternatively, a delivery device can be inserted at the site of administration to accomplish blunt dissection and the cells or the cell-containing flowable composition administered as the delivery device is retracted from the newly created planar administration area

According to an alternative exemplary delivery method, the cells or the cell-containing flowable composition can be administered by manual injection using a penetration device, for example, a needle, guided by ultrasound imaging wherein the penetration device is visible under ultrasound. Exemplary penetration devices include echogenic needles. According to another exemplary delivery method, an echogenic or other material visible under ultrasound is incorporated into the flowable material and the echogenic material is visible under ultrasound during and following administration of the flowable composition to the intended site. The gelatin particle material or the gelatin microcarrier material may be echogenic.

The site of administration may be visualized using ultrasound imaging during administration of the flowable composition and immediately following administration of the flowable composition to evaluate the echogenicity of the penetration device, the echogenicity of the flowable composition, and the efficacy of the echogenic needle and ultrasound guidance device to deliver the cells or the cell-containing flowable composition to the intended site of administration. The site of administration may be visualized using fluoroscopy imaging during and immediately following administration of the flowable composition.

Following administration of the flowable composition according to one of the foregoing exemplary delivery methods, the site of administration can be further analyzed using dissection of the delivery site combined with macroscopic observations. Exemplary observations include measurement of the distance between the delivered flowable composition and the intended delivery site, explantation of any vascular segments and/or surrounding tissue with or without adjacent administered flowable composition, photography of the substance delivery site in *vivo* and/or explanted tissues *ex vivo,* and/or observation of any notable damage to the vasculature or surrounding tissues.

Following administration of the flowable composition, the site of administration can be further analyzed using histology of the site of administration. The portion of the vessel and surrounding tissue, including the delivered flowable composition, can be analyzed. The section of treated vessel is trimmed, fixed in 10% formalin or equivalent, and embedded in paraffin or equivalent. Using approximately 3-6 µm thick sections, five sections will be made through the flowable composition delivery area at 1 mm intervals. The sections will be mounted on gelatin-coated glass slides and stained with an appropriate stain, for example, hematoxylin and eosin or Verhoeff's elastin.

### Example 1: Percutaneous Delivery Applications

Overview: According to one exemplary *in vivo* analysis method, the cells or the cell-containing flowable composition is administered percutaneously to a desired site of administration in a Yorkshire swine model using a needle-based delivery device. According to this method, twenty male or female Yorkshire swine received two femoral stents, one in each of the left and right femoral arteries, following balloon-inflation injury to the treatment site. Nine of the subjects received subsequent percutaneous injection of the cell-containing flowable composition perivascularly to the femoral sites after stent implantation; nine of the subjects received a cell-free flowable composition perivascularly to the femoral sites after stent implantation; two subjects received stents, but received no flowable composition. During percutaneous administration of the cell-containing or cell-free flowable composition, the needle or catheter is directed to the desired site of administration and a sufficient volume of the cells or the cell-containing flowable composition is delivered at the desired perivascular site of administration at or adjacent to the location of the femoral stent. The animal is allowed to recover. The site of administration is subsequently visualized and/or dissected to determine the efficacy of the delivery device and/or the delivery method to deliver the cells or the cell-containing flowable composition to the intended site of administration and/or to determine the efficacy of the administered composition to treat the site in need of treatment.

Protocol: According to the *in vivo* study protocol, prior to and following treatment, the subjects received daily aspirin and clopidogrel to prevent or reduce the occurrence of thrombotic events. After introduction of anesthesia, an incision was made in the neck to expose the carotid artery. An arterial sheath was introduced and advanced into the artery. Heparin was administered after placement of the introducer sheath to prolong activated clotting time to a target range of approximately 275 seconds during device deployment.

Under fluoroscopic guidance, a guide catheter was advanced through the sheath over a guidewire to the right and left femoral arteries. Angiographic images of the vessels were obtained with contrast media to identify the proper location for the deployment site. The right and left arteries were injured by approximately 30-second balloon inflations at a pressure of approximately 10 atmospheres. Each subject received approximately three inflations per side in overlapping segments.

A guidewire was inserted into the targeted femoral artery. Herculink stents (5.5 mm) were implanted. The stent was introduced into the artery by advancing the stent delivery system through the guide catheter and over the guidewire to the deployment site. The balloon was then inflated at a steady rate to a pressure sufficient to target a visually assessed balloon-to-artery ratio and held for approximately 30 seconds from initiation of inflation. After the inflation period is over, vacuum was applied to the inflation device to deflate the balloon. Complete balloon inflation was verified with fluoroscopy. The delivery system was then slowly removed. Contrast injections were used to determine device patency and additional acute deployment characteristics. The procedure was then repeated for the contralateral vessel.

After completion of angiography, the femoral arteries were treated as follows. Dosages of the cell-containing flowable composition and the cell-free flowable composition suspended in media (Endothelial Cell Basal Media, EBM-2, supplemented with 5% FBS and 50 µg/ml gentamicin) were rinsed in a syringe twice with approximately 19 mL of sterile saline (USP) per rinse at room temperature. The flowable composition was then concentrated in saline to 2-3 mL (approximately 30 mg/mL) before injection at the treatment sites. Each dosage of the cell-containing flowable composition contained approximately 3.0 x 10⁴ Porcine Aortic Endothelial (PAE) cells/mg at a density of approximately 30 mg/mL. Each dosage of the cell-free flowable composition contained approximately 70 mg biocompatible substrate particles at a density of approximately 30 mg/mL.

Dosages of the cell-containing or cell-free flowable composition were delivered to the perivascular site of the stented femoral arteries percutaneously using a syringe and hypodermic needle (approximately 18 gauge). Delivery of the composition was visualized by ultrasound guided needle placement techniques using echogenic needles.

Target locations for injection were into or on top of the femoral sheath. Delivery to the treatment sites without vessel puncture was confirmed by visualization of blood flow post-injection by color-Doppler ultrasound or spectral-Doppler ultrasound scan. The right and left femoral arteries were treated with one percutaneous injection of either 70 mg cell-free flowable composition in approximately 2-3 mL saline or injection of cell-containing flowable composition containing approximately 2 x 10⁶ cells per treatment site for a total cell delivery of approximately 4 x 10⁶ cells per subject. The total cell load based on body weight was approximately 1.4 x 10⁵ cells per kilogram. Two animals in the sham group received no treatment. When complete, the carotid access wound was closed in layers and the animal was allowed to recover from anesthesia.

To confirm consistent delivery expansion of each stent, quantitative vascular angiography was performed for each stented vessel at at least four time points: on Day 0 before device implantation; on Day 0 during device/balloon deployment; on Day 0 after device deployment; and before euthanasia/necropsy. Clinical observations were performed at least once daily and catheter access sites were observed daily for 10-14 days after intervention to ensure proper healing. Blood was collected from a peripheral vein for clinical pathology analysis, including hematology, clinical chemistry, coagulation and ACT, at baseline, 5-10 minutes post heparin administration, and at 4, 8 and 12 weeks post-treatment. Hematology analysis included at least the following parameters: total leukocyte count (WBC); erythrocyte count (RBC); hemoglobin concentration (HGB); hematocrit value (HCT); mean corpuscular volume (MCV); mean corpuscular hemoglobin (MCH); mean corpuscular hemoglobin concentration (MCHC); platelet count (PLT); and blood smear evaluation (including differential). Serum clinical chemistry analysis included at least the following parameters: glucose (GLU); urea nitrogen (BUN); creatinine (CRE); total protein (TPR); albumin (ALB); globulin (GLOB); albumin/globulin ratio (A/G); calcium (CAL); phosphorus (PHOS); electrolytes (NA, K, CL); total cholesterol (CHOL); total bilirubin (TBIL); triglycerides (TRG); alanine aminotransferase (ALT); aspartate aminotransferase (AST); alkaline phosphatase (ALK); and gamma glutamyltransferase (GGT). Coagulation analysis included at least the following parameters: prothrombin time (PT); activated partial thromboplastin time (APTT); and fibrinogen (FIB).

Prior to sacrifice, each treatment site was visualized using angiography of the treatment site and of sites both proximal and distal to the treatment site. Patency and degree of stenosis of femoral arteries was determined by blinded analysis of the angiograms in paired comparison with baseline angiograms. Ultrasound imaging was also performed, as needed.

Following sacrifice, the femoral arteries were perfusion fixed at approximately 100 mmHg with lactated Ringer's solution, until cleared of blood, followed by 10% neutral buffered formalin (NBF). The fixed vessels and associated surrounding tissue including, at least, adventitial/femoral sheath tissue, were carefully dissected free, ensuring collection of injection sites of flowable composition, as well as unstented vessel both proximal and distal to the stented portion. In addition, associated lymph nodes, including iliac lymph nodes, were collected.

All stented and unstented sections of vessels, surrounding perivascular tissues including up to 2-cm segments beyond the stent, were processed for histology according to standard histology procedures. For each vessel, sections along the length of the stent were analyzed as follows: 1 section of each nonstented vessel, located within 1-3 mm of both proximal and distal ends of the stent in nonstented tissue; and 3 sections from each stented vessel taken from the proximal end, mid-portion and distal end. Two slides were cut at each level and stained with hematoxylin and eosin and tissue elastin stains, for example, Verhoeff's. All stained slides were examined and scored in blinded fashion by a board certified Veterinary Pathologist. In addition, all associated lymphoid tissue was processed and assessed microscopically.

Quantitative histomorphometric analysis was conducted on the histological sections from each stented artery. For each histological section, lumen area, internal elastic layer (IEL) bounded area, stented area, and external elastic layer (EEL) bounded area were directly measured using standard light microscopy and computer-assisted image measurement systems. Histopathological scoring via light microscopy was used to grade various parameters that reflect the degree and extent of the host response/repair process to the treatment. These parameters were divided between those associated with the stent and vessel (i.e., observations of the intima, media and adventitia) and those associated with the tissue reactivity to the injected material (i.e., observations of the perivascular tissue). Any remaining injected flowable composition present at the time of sacrifice was located macroscopically at the time of necropsy and microscopically on stained slides and evaluated for distribution along the vessel and into the surrounding tissue.

To evaluate the effect of the flowable composition on the vessel lumen, scoring of the response/repair process of the vessel to the stents included, but was not limited to, injury, inflammation, endothelialization, and fibrin deposition. Injury score for stented arterial segments depended on the part of the arterial wall perturbed by the stent. Injury was scored on a per-strut basis and the average calculated per plane and stent. Inflammation was scored based on the degree and extent of inflammation on a per-strut basis and the average calculated per plane and stent. The intimal fibrin score depended on the degree of intimal fibrin deposition and the relative extent of the circumstances of the artery involved. The endothelialization score depended on the extent of the circumference of the artery lumen showing coverage with endothelial cells. The histological sections of the stented arteries as well as sections of the adventitia and perivascular tissue were also examined for other histological parameters including, but not limited to: hemorrhage, necrosis, medial fibrosis, type and relative amounts of inflammatory cell infiltrates (e.g., neutrophils, histiocytes, lymphocytes, and multinucleated giant cells), cellularity of the neointima, or other parameters deemed appropriate by the pathologist.

Results: All of the femoral arteries were patent at the one-month time point. Angiographic analysis performed on pigs four weeks post-injury revealed stenosis in the stented segments of control animals. Animals that received the cell-containing flowable composition displayed reduced percent stenosis of the stented arteries by 55% (P<0.05) compared to cell-free flowable composition. The cell-containing flowable composition also significantly reduced the average and worst percent stenosis compared to the cell-free flowable composition.

Detailed angiographic analysis at four weeks post-injury revealed an increase in stenosis in the stented segments of the vessels in animals that received injections into the adjacent muscle capsule compared to those who received injections into or on top of the femoral sheath. Arteries treated with the cell-containing flowable composition injected into or on top of the femoral sheath had a percent stenosis of 17.2 ± 4.1 and 13.9 ± 2.5, respectively. In comparison, arteries treated with the cell-containing flowable composition injected into the adjacent muscle had a percent stenosis of 26.7 ± 3.5 (P<0.05). The area of maximal stenosis identified angiographically agreed with that observed upon histological assessment and corresponded to the proximal segment of the stented vessels. Analysis of the proximal planes, where the greatest response appeared to be present, also revealed significantly greater intimal area (P<0.05) and thickness in the animals injected with the cell-containing flowable composition into the adjacent muscle compared to those injected with the cell-containing flowable composition into or on top of the femoral sheath.

The levels of inflammation and fibrin deposition associated with the lumen were scored and found to be low in all treatment groups evaluated. Inflammation scores were semi-quantitative measures of the extent of local arterial wall inflammation and each stent strut was scored according to the degree of inflammation. Fibrin deposition in the intima is a characteristic response seen in drug-eluting stents and is not expected with bare metal stents. All treatment groups exhibited similar levels of fibrin deposition and were representative of bare metal stents at four weeks. The inflammatory and fibrotic response associated with the adventitia, femoral sheath and muscle sheath were also evaluated. Overall, there was very little inflammatory response associated with the adventitia, femoral sheath or the adjacent muscle fascia. The majority of the inflammatory cell types associated with the adventitia and femoral sheath consisted of histocytes and lymphocytes. Arteries treated with cell-containing flowable composition injected on top of the femoral sheath had the least amount of lymphocytes in the adventitia and femoral sheath when compared to the other groups. Adventitial fibrosis was also present in all treatment groups and was generally minimal, however, adventitial fibrosis tended to be the least severe in arteries treated with cell-containing flowable composition injected on top of the femoral sheath.

| Characteristics | Composition Injected Into Femoral Sheath | Composition Injected On Top of Femoral Sheath | Composition Injected Into Adjacent Muscle |
|---|---|---|---|
| Number of arteries, *n* | 6 | 6 | 6 |
| Intima Area (mm²) | 3.50 ± 0.40 | 3.85 ± 0.22 | 4.68 ± 0.45 |
| • Proximal | 4.05 ± 0.56 | 4.75 ± 0.53 | 6.35 ± 0.95* |
| Intimal Thickness (mm) | 0.27 ± 0.08 | 0.27 ± 0.03 | 0.35 ± 0.10 |
| • Proximal | 0.31 ± 0.12 | 0.33 ± 0.08 | 0.46 ± 0.20 |
| Media Area (mm²) | 2.21 ± 0.14 | 2.20 ± 0.12 | 2.38 ± 0.16 |
| • Proximal | 2.49 ± 0.33 | 2.35 ± 0.18 | 2.56 ± 0.13 |
| Lumen Area (mm²) | 12.51 ± 0.67 | 14.5 ± 0.63 | 12.58 ± 1.11 |
| • Proximal | 12.38 ± 0.91 | 13.90 ± 0.34 | 12.57 ± 1.17 |
| Average % Stenosis | 22.0 ± 2.5 | 21.0 ± 0.41 | 28.0 ± 3.3 |
| • Proximal | 25 ± 3.70 | 25 ± 2.05 | 34 ± 5.33 |

The results of the above-described study suggest that perivascular endothelial cells promote vascular repair after stent-induced injury and cells delivered on top of the femoral sheath provided similar benefit to cells delivered into the femoral sheath. Moreover, cells delivered outside the femoral sheath appeared to have a lower incidence of adventitial lymphocytes and fibrosis compared to cells delivered inside the femoral sheath, although they were equally effective in suppressing intimal formation and stenosis.

Additional Considerations: Exemplary histology sections of a femoral artery and a superficial femoral artery were prepared following perivascular administration of the cell-containing flowable composition. In each of these exemplary sections, the flowable composition was administered percutaneously with the assistance of ultrasound guidance according to the method described above. The histology sections indicate that the flowable composition was delivered and resides within the femoral sheath but external to the wall of the treated blood vessel. Further, the flowable composition resides at each of the sites of administration in an acceptable concentration and does not spread or disburse to an unacceptable degree.

### Example 2: Anatomical Location Applications

Overview: To evaluate the safety and efficacy of perivascular injections of the flowable composition at different locations peripheral to stented femoral arteries, the flowable composition was administered to a variety of non-luminal anatomical locations adjacent to the treatment site in a Yorkshire swine model, The cell-containing flowable composition was administered to three anatomical locations: the interior surface of the femoral sheath adjacent the blood vessel (Group 1), the exterior surface of the femoral sheath adjacent the muscular sheath (Group 2), and the interior surface of the muscular sheath adjacent the muscle (Group 3). A control group received an injection of a cell-free flowable composition administered to the exterior surface of the femoral sheath (Group 4). The flowable composition can be administered to additional anatomical locations adjacent the blood vessel including but not limited to the media of the blood vessel, the adventitia of the blood vessel, and the muscle tissue adjacent the blood vessel. The flowable composition can be administered to anatomical locations adjacent other administration sites, including but not limited to structures or spaces adjacent solid organs, tissues or other anatomical locations suitable for treatment with the flowable composition.

Protocol: According to this experimental protocol, cell-containing flowable composition was administered to various anatomical locations and the safety and efficacy of the administration evaluated several days or months following administration. Twelve female Yorkshire domestic swine underwent balloon angioplasty followed by introduction of a biliary stent at two locations in each animal. Right carotid arterial access was obtained using a 7 French sheath. A 5.0-mm-diameter angioplasty balloon was advanced to the left and right femoral arteries under fluoroscopic guidance. The right and left arteries were injured by 30-second balloon inflations at 10 atmospheres pressure; 3 inflations per side in overlapping segments. Biliary stents (6.0-8.0 mm x 18 mm) were introduced into the injured regions of the left and right femoral arteries. Angiography and ultrasound imagery were performed and the stents expanded with the 5.0-mm diameter angioplasty balloon at 10-12 atmospheres pressure. All animals received a bolus of heparin (200 units/kg) at the time of stent deployment.

After final angiography and ultrasound imaging to assess vessel patency, the femoral arteries were treated as follows. The animals were divided into four groups of three, each animal undergoing treatment at two arterial sites. Under fluoroscopic guidance, a guide catheter was advanced through the sheath over a guidewire to the right and left femoral arteries. Angiographic images of the vessels were obtained with contrast media to identify the proper location for the deployment site. The right and left arteries were injured by 30-second balloon inflations at a pressure of 10 atmospheres (approximately 3 inflations per side in overlapping segments). A guidewire was inserted into the targeted femoral artery. Herculink stents (5.0 x 18mm) were implanted. Contrast injections were used to determine device patency and additional acute deployment characteristics. This procedure was then repeated for the contralateral vessel.

Control and test particles suspended in media (Endothelial Cell Basal Media, EBM-2, supplemented with 5% FBS and 50 µlg/mL gentamicin) were rinsed in a syringe with approximately 5 mL of sterile saline at room temperature and concentrated to approximately 30 mg/mL before injection at the treatment site. Sites were not treated with test article until all bleeding was controlled and the area was made as dry as possible.

The femoral arteries were exposed in animals from Groups 1 and 4 prior to injection. The adjacent muscle was exposed (but not the artery) prior to injection in animals from Groups 2 and 3. Each femoral artery of animals in Group 4 was treated via injection with a 20-gauge needle into the femoral sheath with approximately 70 mg of cell-free flowable composition in 2-3 mL sterile saline. Each femoral artery of animals in Groups 1, 2 and 3 was treated with approximately 70 mg of cell-containing flowable composition in 2-3 mL saline. All animals in Group 1 had the cell-containing flowable composition injected into the femoral sheath. Animals in Group 2 had the cell-containing flowable composition injected outside the femoral sheath. Animals in Group 3 had the cell-containing flowable composition injected into the adjacent muscle sheath.

Group 1 received 2-4 mL flowable composition containing approximately 2 x 10⁶ endothelial cells injected into the femoral sheath, adjacent the blood vessel. Group 2 received 2-4 mL flowable composition containing approximately 2 x 10⁶ endothelial cells injected outside the femoral sheath, adjacent the muscular sheath. Group 3 received 2-4 mL flowable composition containing approximately 2 x 10⁶ endothelial cells injected into the muscular sheath, adjacent the muscle. Group 4 received control cell-free particulate biocompatible matrix material injected into the femoral sheath, adjacent the blood vessel.

Following administration of the flowable composition or the control composition, the administration sites were digitally photographed. The access wound was closed in layers and the animal was allowed to recover from anesthesia. At day 28, pre-sacrifice angiography and ultrasound imaging, both longitudinal and transverse, were performed for comparison to baseline and quantitative analysis was performed. Access to the target site was via carotid cutdown. An arterial sheath was introduced and advanced into the artery. Heparin (200 U/kg) was administered. Using fluoroscopic guidance, an appropriately-sized angiographic catheter was inserted through the sheath and advanced to the site of device deployment. Angiograms that included the vessels both proximal and distal to the device were obtained in the same views as at the time of implants. Quantitative measurements were manually recorded for each individual device.

A limited necropsy, defined as a macroscopic examination of the external surface of the body and microscopic examination of the femoral sites was performed on all animals at 28 days. All stented and unstented sections of vessels and surrounding perivascular tissues and muscle tissues (including 2 mm to 2 cm segments beyond the stent) were processed according to standard histology procedures. For each vessel, sections along the length of the stent were analyzed as follows: 1 section each of nonstented vessel, located within 1-3 mm of both the proximal and distal ends of the stent in nonstented tissue, and 3 sections from each stented vessel taken from the proximal end, mid-portion and distal end. Two slides were cut at each level and stained with hematoxylin and eosin and Verhoeff's tissue elastin stains.

Quantitative morphometric analysis was performed on the histological sections from each stented artery. For, each histological section, lumen area, internal elastic layer (IEL) bounded area, stent area, and external elastic layer (EEL) bounded area were directly measured using standard light microscopy and computer-assisted image measurement systems.

Histopathological scoring was also used to grade various parameters that reflect the degree and extent of injury, and inflammation, as well as the host response to the treatment and the repair process. These parameters include injury, inflammation, endothelialization, and fibrin deposition. The scores generally reflected the degree and extent of injury. Scoring was performed using light microscopy. Injury score for stented arterial segments depended on the part of the arterial wall perturbed by the stent. Injury was scored on a per-strut basis and the average calculated per plane and stent. Inflammation was scored based on the degree and extent of inflammation on a per-strut basis and the average calculated per plane and stent. The intimal fibrin score depended on the degree of intimal fibrin deposition and the relative extent of the circumstances of the artery involved. The endothelialization score depended on the extent of the circumference of the artery lumen showing coverage with endothelial cells. The histological sections of the stented arteries as well as sections of the adventitia and perivascular tissue were also examined for other histological parameters including, but not limited to: hemorrhage, necrosis, medial fibrosis, type and relative amounts of inflammatory cell infiltrates (e.g., neutrophils, histiocytes, lymphocytes, and multinucleated giant cells), cellularity of the neointima, or other parameters deemed appropriate by the pathologist.

Results: All subjects survived to the designated one-month end point and there were no mortalities. Angiography, clinical observations and necropsy were performed at the protocol specified time points. All animals enrolled into the study successfully completed the study as outlined in the protocol. All clinical observations, macroscopic and microscopic pathology findings were similar between groups. All stented femoral arteries were patent at the one-month time point. There was a slight decrease in percent stenosis in arteries injected with cell-containing flowable composition into the femoral sheath (Group 1: 16.6%) or outside the femoral sheath (Group 2: 13.9%) when compared to arteries injected with test article into the adjacent muscle (Group 3: 22%) or with control article into the femoral sheath (Group 4: 22), as determined by quantitative vascular angiography.

Microscopically, there were no statistically significant differences in neointimal thickness or intimal area as whole vessel averages or by proximal, mid and distal planes. Overall, the reactive smooth muscle cell hyperplastic response was considered to be mild for all treatment groups. However, a few trends were noted in the proximal plane where the greatest response appeared to be present in all groups. Neointimal thickness appeared to be greatest in Groups 3 and 4 (0.463 and 0.377 mm, respectively) compared to Groups 1 and 2 (0.306 and 0.333 mm, respectively). Similarly, neointimal area appeared to be greatest in Groups 3 and 4 (6.347 and 5.43 mm², respectively) compared to Groups 1 and 2 (4.04 and 4.75 mm², respectively). Both mechanical injury and inflammation were low in this study. A statistical difference was found in artery area, which may be attributed to statistically significant differences in stent diameters deployed at the time of implantation as measured histomorphometrically and via quantitative vascular angiography. There did not appear to be negative remodeling associated with the vascular response in this study.

Semi-quantitative histomorphometric analysis was performed to assess inflammation, injury, fibrin deposition, and re-endothelialization, with no statistical differences observed between treatment groups, Adventitial inflammation and fibrosis was also minimal, with the lowest incidence of lymphocytes and fibrosis in Group 2 animals.

The injection of flowable composition, regardless of injection site, did not cause delayed healing as all treatment groups exhibited near complete re-endothelialization at one month. Overall, injections of flowable composition either into or adjacent to the stented femoral sheath appeared to have the greatest potential benefit compared to injections into the adjacent muscle.

### Example 3: Cell Dosage

To evaluate the safety and efficacy of different dosages of the flowable composition administered to a desired site of administration, the flowable composition will be administered in a variety of dosages or cell numbers. The flowable composition will be administered in three different concentrations or cell counts at the same anatomical location, for example, within the femoral sheath adjacent the blood vessel: 0.5x10⁶ cells in 0.5 mL biocompatible material; 1x10⁶ cells in 1 mL biocompatible material; and 2x10⁶ cells in 2 mL biocompatible material.

Flowable composition will be administered in various dosages and the safety and efficacy of the administration evaluated several days or months following administration. In one procedure twelve male or female Yorkshire domestic swine will undergo balloon angioplasty followed by introduction of a biliary stent at two locations in each animal. Right carotid arterial access will be obtained using a 7 French sheath. A 5.0-mm-diameter angioplasty balloon will be advanced to the left and right femoral arteries under fluoroscopic guidance. The right and left arteries will be injured by 30-second balloon inflations at 10 atmospheres pressure; 3 inflations per side in overlapping segments. Biliary stents (6.0-8.0 mm x 18 mm) will be introduced into the injured regions of the left and right femoral arteries. Angiography and ultrasound imagery will be performed and the stents expanded with the 5.0-mm diameter angioplasty balloon at 10-12 atmospheres pressure. All animals will receive a bolus of heparin (200 units/kg) at the time of stent deployment.

After final angiography and ultrasound imaging to assess vessel patency, the femoral arteries will be treated as follows. The animals will be divided into four groups of three. The first group will receive 0.5 mL flowable composition containing approximately 0.5 x 10⁶ endothelial cells injected into the femoral sheath, adjacent the blood vessel. The second group will receive 1 mL flowable composition containing approximately 1 x 10⁶ endothelial cells injected into the femoral sheath, adjacent the blood vessel. The third group will receive 2 mL flowable composition containing approximately 2 x 10⁶ endothelial cells injected into the femoral sheath, adjacent the blood vessel. The fourth group will receive control particulate biocompatible matrix material injected into the femoral sheath, adjacent the blood vessel.

Following administration of the flowable composition or the control composition, the animal will be allowed to recover from anesthesia. At day 28, pre-sacrifice angiography and ultrasound imaging, both longitudinal and transverse, will be performed for comparison to baseline and quantitative analysis will be performed. A limited necropsy, defined as a macroscopic examination of the external surface of the body and microscopic examination of the femoral sites will be performed on all animals at 28 days. The femoral sites and surrounding tissues will be excised and stained with Verhoeff's elastin for histopathological and morphometric evaluation.

### Example 4: Endoluminal Administration

To evaluate the safety and efficacy of perivascular administration of the flowable composition administered to a desired site of administration using an endoluminal route of administration, the flowable composition will be administered using a minimally invasive catheter and needle system. In one procedure, three groups of animals will, for example, receive an administration of the flowable composition injected from the lumen into the perivascular space of a stented femoral artery; an administration of control biocompatible material without cells from the lumen into the perivascular space of a stented femoral artery; and sham treated or no administration of material, but following stenting of the femoral artery.

According to these procedures, flowable composition will be administered using a minimally invasive catheter-needle system and the safety and efficacy of the administration evaluated several days or months following administration. Twelve male or female Yorkshire domestic swine will undergo balloon angioplasty followed by introduction of a biliary stent at two locations in each animal. Right carotid arterial access will be obtained using a 7 French sheath. A 5.0-mm-diameter angiplasty balloon will be advanced to the left and right femoral arteries under fluoroscopic guidance. The right and left arteries will be injured by 30-second balloon inflations at 10 atmospheres pressure; 3 inflations per side in overlapping segments. Biliary stents (6.0-8.0 mm x 18 mm) will be introduced into the injured regions of the left and right femoral arteries. Angiography and ultrasound imagery will be performed and the stents expanded with the 5.0-mm diameter angioplasty balloon at 10-12 atmospheres pressure. All animals will receive a bolus of heparin (200 units/kg) at the time of stent deployment.

After final angiography and ultrasound imaging to assess vessel patency, the femoral arteries will be treated as follows. The animals will be divided into three groups of three. The first group will receive 2 mL flowable composition containing approximately 2 x 10⁶ endothelial cells injected from the lumen into the perivascular space of the blood vessel. The second group will receive 2 mL control biocompatible material injected from the lumen into the perivascular space of the blood vessel. The third group will undergo sham angioplasty and stenting, but will not receive either the flowable composition or the control biocompatible material.

Following administration of the flowable composition or the control composition, the animal will be allowed to recover from anesthesia. At day 28, pre-sacrifice angiography and ultrasound imaging, both longitudinal and transverse, will be performed for comparison to baseline and quantitative analysis will be performed. A limited necropsy, defined as a macroscopic examination of the external surface of the body and microscopic examination of the femoral sites will be performed on all animals at 28 days. The femoral sites and and surrounding tissues will be excised and stained with Verhoeff's elastin for histopathological and morphometric evaluation.

### Example 5: Dispensing Container Evaluation

To evaluate the effectiveness of the dispensing container 50 described above, experimentation was conducted to determine the effect on the flowable composition of the rinse procedure and the transfer procedure. In particular, rinse procedure experimentation was designed to evaluate the efficiency of removal of media from the material during the rinse procedure and the effect on cell viability and functionality, of the rinse procedure and various rinse solutions. Material transfer experimentation was designed to evaluate the efficiency of transfer of the material out of the dispensing container and the effects on the cells of the material, including viability and functionality, of the transfer procedure.

To evaluate the rinse procedure, cell counts were conducted following the rinse procedure, outlined above in the description of the dispensing container, using three rinse solutions: saline, Lactated Ringer's Solution and Human Serum Albumin in saline. Lactated Ringer's Solution was found to result in less cell loss during rinse, compared to saline. Supplementing the saline fluid with Human Serum Albumin similarly was found to result in less cell loss during rinse, compared to saline. Combining Lactated Ringer's Solution and Human Serum Albumin improved cell survival during rinse more than either alone. The immediate and recovery cell counts using each rinse solution are provided below:

| | Immediate Count | | Recovery Count | |
|---|---|---|---|---|
| | Average Viable Cells | Average Viability | Average Viable Cells | Average Viability |
| Control | 2.71E+06 | 85.5% | 2.36E+06 | 83.8% |
| Saline | 9.14E+05 | 84.2% | 8.02E+05 | 84.2% |
| HSA in Saline | 1.92E+06 | 87.3% | 1.38E+06 | 86.2% |
| Ringer's | 1.38E+06 | 82.2% | 1.35E+06 | 85.3% |

To further evaluate the rinse procedure, cell viability was evaluated using assays for heparin sulfate (HS), TGF-β and bFGF, following the rinse procedure outlined above in the description of the dispensing container, using three rinse solutions: saline, Lactated Ringer's Solution and Human Serum Albumin in Lactated Ringer's Solution. Lactated Ringer's Solution was found to result in acceptable cell functionality following rinse, compared to saline alone (results not shown). Results are provided below:

| | HS (µg/mL) | TGF-β (pg/mL) | bFGF (pg/mL) |
|---|---|---|---|
| 0.25% HSA in Ringer's, Sample 1 | 0.36 | 564 | 226 |
| 0.25% HSA in Ringer's, Sample 2 | 0.37 | 480 | 181 |
| 0.25% HSA in Ringer's, Sample 3 | 0.65 | 561 | 235 |
| 0.25% HSA in Ringer's, Average | 0.46 | 535 | 214 |
| No Treatment Control | 1.08 | 703 | 205 |
| Release Criteria for Composition | >0.23 | >300 | FIO |

## Claims

1. A device (50) for storing and rinsing, or delivering, a flowable composition comprising cells, the device comprising: a first chamber (72); a second chamber (102) in fluid communication with the first chamber; a plunger (106) comprising a screen (96) positioned between the first chamber and the second chamber, wherein the screen permits passage of fluid and denies passage of the flowable composition from the first chamber to the second chamber.

2. The device of claim 1 for delivering a flowable composition, the device further comprising a penetration device (62), wherein the penetration device (62) is in fluid communication with the first chamber (72).

3. The device of claim 2 wherein the penetration device (62) comprises an internal diameter of 18 gauge to 24 gauge.

4. The device of claim 1 for storing and rinsing a flowable composition, the device further comprising a third chamber in fluid communication with the first chamber, wherein the third chamber comprises a fluid.

5. The device of claim 4 wherein the fluid comprises saline and, optionally, further comprises at least one of Human Serum Albumin and Lactated Ringer's Solution

6. The device of claim 4 or claim 5 wherein the device is adapted to pass the fluid from the third chamber, through the first chamber (72), through the screen (96) and into the second chamber.

7. The device of any of the preceding claims wherein the first chamber (72) is adapted to collect flowable composition comprising cells and permit excess fluid to flow into the second chamber (102).

8. The device of any of the preceding claims wherein the plunger (106) is adapted to move from the second chamber (102) into the first chamber (72).

9. The device of any of the preceding claims wherein the first chamber further comprises cells and wherein the device is adapted to rinse the cells contained within the first chamber (72) with a fluid, to retain the cells within the first chamber (72), and to collect the fluid in the second chamber (102).

10. The device of any of the preceding claims further comprising the flowable composition, wherein the flowable composition comprises a biocompatible matrix and cells and wherein the cells are associated via cell to matrix interactions with the biocompatible matrix.

11. The device of claim 10 wherein the biocompatible matrix comprises microcarriers having a diameter of about 20 microns to about 500 microns.

12. The device of claim 11 wherein the microcarriers have a diameter of about 200 microns.

13. The device of claims 11 or claim 12 wherein the microcarriers comprise gelatin.

14. The device of claim 13 wherein the microcarriers comprise hydrated CultiSpher-G™ microcarrier beads.

15. The device of any one of claims 9 to 14 wherein the cells are endothelial, endothelial-like or non-endothelial cells.

## Patentansprüche

1. Eine Vorrichtung (50) zum Speichern und Spülen oder Ausbringen einer zellhaltigen fließfähigen Zusammensetzung, wobei die Vorrichtung umfasst: eine erste Kammer (72); eine zweite Kammer (102) in Flüssigkeitsverbindung mit der ersten Kammer (72); einen Kolben (106) umfassend eine Dichtungswand (96), die zwischen der ersten Kammer und der zweiten Kammer positioniert ist, wobei die Dichtungswand die Passage von Flüssigkeit erlaubt und die Passage der fließfähigen Zusammensetzung von der ersten Kammer zur zweiten Kammer verwehrt.

2. Die Vorrichtung nach Anspruch 1 zum Ausbringen einer fließfähigen Zusammensetzung, wobei die Vorrichtung weiterhin eine Penetrationsvorrichtung (62) umfasst, wobei die Penetrationsvorrichtung (62) in Flüssigkeitsverbindung mit der ersten Kammer (72) steht.

3. Die Vorrichtung nach Anspruch 2, wobei die Penetrationsvorrichtung (62) einen inneren Durchmesser von 18 bis 24 Gauge umfasst.

4. Die Vorrichtung nach Anspruch 1 zum Speichern und Spülen einer fließfähigen Zusammensetzung, wobei die Vorrichtung weiterhin eine dritte Kammer umfasst, die in fließender Verbindung mit der ersten Kammer steht, wobei die dritte Kammer eine Flüssigkeit enthält.

5. Die Vorrichtung nach Anspruch 4, wobei die Flüssigkeit isotonische Kochsalzlösung umfasst und optional weiterhin wenigstens eines von Humanalbumin und Ringer-Lactat-Lösung.

6. Die Vorrichtung nach Anspruch 4 oder 5, wobei die Vorrichtung angepasst ist, um die Flüssigkeit von der dritten Kammer durch die erste Kammer (72), durch die Dichtungswand (96) und in die zweite Kammer zu führen.

7. Die Vorrichtung nach einem der vorangegangenen Ansprüche, wobei die erste Kammer (72) angepasst ist, um eine zellhaltige fließfähige Zusammensetzung zu sammeln und den Fluss überschüssiger Flüssigkeit in die zweite Kammer (102) zu erlauben.

8. Die Vorrichtung nach einem der vorangegangenen Ansprüche, wobei der Kolben (106) angepasst ist, um sich von der zweiten Kammer (102) in die erste Kammer (72) zu bewegen.

9. Die Vorrichtung nach einem der vorangegangenen Ansprüche, wobei die erste Kammer weiterhin Zellen umfasst und wobei die Vorrichtung angepasst ist, die in der ersten Kammer (72) enthaltenen Zellen mit Flüssigkeit zu spülen, die in der ersten Kammer (72) enthaltenen Zellen zurückzuhalten, und die Flüssigkeit in der zweiten Kammer (102) zu sammeln.

10. Die Vorrichtung nach allen vorangegangenen Ansprüchen weiterhin die fließfähige Zusammensetzung umfassend, wobei die fließfähige Zusammensetzung eine biokompatible Matrix und Zellen umfasst, und wobei die Zellen über Zelle-Matrix-Interaktionen mit der biokompatiblen Matrix verbunden sind.

11. Die Vorrichtung nach Anspruch 10, wobei die biokompatible Matrix Mikroträger umfasst, die einen Durchmesser von etwa 20 Mikrometer bis 500 Mikrometer haben.

12. Die Vorrichtung nach Anspruch 11, wobei die Mikroträger einen Durchmesser von etwa 200 Mikrometer haben.

13. Die Vorrichtung nach Anspruch 11 oder 12, wobei die Mikroträger Gelatine umfassen.

14. Die Vorrichtung nach Anspruch 13, wobei die Mikroträger hydratisierte CultiSpher-G^{™} - Mikroträgerkügelchen umfassen.

15. Die Vorrichtung nach einem der Ansprüche 9 bis 14, wobei die Zellen Endothelzellen, endothelzellähnliche Zellen oder Nicht-Endothelzellen sind.

## Revendications

1. Dispositif (50) permettant de stocker et rincer, ou délivrer, une composition écoulable comprenant des cellules, le dispositif comprenant : une première chambre (72) ; une deuxième chambre (102) en communication fluidique avec la première chambre ; un plongeur (106) comprenant un tamis (96) positionné entre la première chambre et la seconde chambre, où le tamis permet le passage de fluide et refuse le passage de la composition écoulable de la première chambre à la deuxième chambre.

2. Dispositif selon la revendication 1, permettant de délivrer une composition écoulable, le dispositif comprenant en outre un dispositif de pénétration (62), où le dispositif de pénétration (62) est en communication fluidique avec la première chambre (72).

3. Dispositif selon la revendication 2, dans lequel le dispositif de pénétration (62) comprend un diamètre interne de calibre 18 à calibre 24.

4. Dispositif selon la revendication 1, permettant de stocker et rincer une composition écoulable, le dispositif comprenant en outre une troisième chambre en communication fluidique avec la première chambre, où la troisième chambre comprend un fluide.

5. Dispositif selon la revendication 4, dans lequel le fluide comprend du sérum physiologique et, facultativement, comprend en outre au moins un élément parmi l'albumine de sérum humain et la solution de Ringer lactate.

6. Dispositif selon la revendication 4 ou la revendication 5, dans lequel le dispositif est adapté pour faire passer le fluide de la troisième chambre à la deuxième chambre en passant par la première chambre (72) et le tamis (96).

7. Dispositif selon l'une quelconque des revendications précédentes, dans lequel la première chambre (72) est adaptée pour collecter la composition écoulable comprenant des cellules et permettre à un excès de fluide de s'écouler dans la deuxième chambre (102).

8. Dispositif selon l'une quelconque des revendications précédentes, dans lequel le plongeur (106) est adapté pour se déplacer de la deuxième chambre (102) dans la première chambre (72).

9. Dispositif selon l'une quelconque des revendications précédentes, dans lequel la première chambre comprend en outre des cellules et dans lequel le dispositif est adapté pour rincer les cellules contenues dans la première chambre (72) avec un fluide, pour retenir les cellules dans la première chambre (72), et pour collecter le fluide dans la deuxième chambre (102).

10. Dispositif selon l'une quelconque des revendications précédentes, comprenant en outre la composition écoulable, dans lequel la composition écoulable comprend une matrice biocompatible et des cellules et dans lequel les cellules sont associées via des interactions cellule-matrice avec la matrice biocompatible.

11. Dispositif selon la revendication 10, dans lequel la matrice biocompatible comprend des microvecteurs ayant un diamètre d'environ 20 microns à environ 500 microns.

12. Dispositif selon la revendication 11, dans lequel les microvecteurs ont un diamètre d'environ 200 microns.

13. Dispositif selon la revendication 11 ou la revendication 12, dans lequel les microvecteurs comprennent de la gélatine.

14. Dispositif selon la revendication 13, dans lequel les microvecteurs comprennent des billes de microvecteur de CultiSpher-G™ hydratés.

15. Dispositif selon l'une quelconque des revendications 9 à 14, les cellules sont des cellules endothéliales, de type endothélial ou non endothélial.
